# FASCICULE DE BREVET EUROPEEN

(11) **EP 3 066 199 B1**
(45) Date de publication et mention de la délivrance du brevet: **21.03.2018**
(21) Numéro de dépôt: 14796053.8
(22) Date de dépôt: 07.11.2014
(51) Int. Cl.: C12N 15/113, A61K 31/7105

(54) **SNO ARN, COMPOSITIONS ET UTILISATIONS**
SNORA, ZUSAMMENSETZUNGEN UND VERWENDUNG
SNORNA, COMPOSITIONS AND USES

(30) Priorité: 07.11.2013 FR 1360889
(43) Date de publication de la demande: 14.09.2016
(73) Titulaire: NINOVAX, 75008 Paris (FR)
(72) Inventeur: Martin, Jean-René, 91400 Orsay (FR)
(74) Mandataire: Cabinet Becker et Associés
(86) Numéro de dépôt international: PCT/EP2014/073991
(87) Numéro de publication internationale: WO 2015/067727

(56) Documents cités:
- Z.-P. HUANG ET AL: "Genome-wide analyses of two families of snoRNA genes from Drosophila melanogaster, demonstrating the extensive utilization of introns for coding of snoRNAs", RNA, vol. 11, no. 8, 1 août 2005 (2005-08-01), pages 1303-1316, XP055058371, ISSN: 1355-8382, DOI: 10.1261/rna.2380905 cité dans la demande -& DATABASE EMBL [Online] 3 février 2005 (2005-02-03), "Drosophila melanogaster psi28s-1153 snoRNA gene", XP055166324, extrait de EBI accession no. EMBL:AJ809559 Database accession no. AJ809559
- MASAOMI KATO ET AL: "Ageing and the small, non-coding RNA world", AGEING RESEARCH REVIEWS, vol. 12, no. 1, 6 avril 2012 (2012-04-06), pages 429-435, XP055058373, ISSN: 1568-1637, DOI: 10.1016/j.arr.2012.03.012
- HUAQI JIANG ET AL: "Intestinal stem cells in the adultmidgut", EXPERIMENTAL CELL RESEARCH, ACADEMIC PRESS, US, vol. 317, no. 19, 23 juillet 2011 (2011-07-23), pages 2780-2788, XP028103415, ISSN: 0014-4827, DOI: 10.1016/J.YEXCR.2011.07.020 [extrait le 2011-08-11]

### SEQUENCE LISTING

<110> Centre National de la Recherche Scientifique
<120> SNO ARN Compositions & Utilisations
<130> B1365PC00
<160> 37
<170> PatentIn version 3.5
<210> 1
   <211> 148
   <212> RNA
   <213> Drosophila melanogaster
<400> 1
   aaagcguuag auauuaaacu gugguugaau ucacaaaaua ggccacaguu augcaauaaa 60
   cgcuagaaaa aaaacgguag uauuuaauaa cguguugacu aacaucugcg gauaagaagc 120
   uuugcguuuu gagguacuaa ccacagua 148
<210> 2
   <211> 159
   <212> RNA
   <213> Homo sapiens
<400> 2
   guaaguguag ccuagaaauu ggggcuggau uugaaaauua gccccaauuc ugcaauuuuc 60
   accgcaauaa aagcuucucc aguuauacau ggugauuggu cuugaugggc uauuguggac 120
   agaggagggu gcuagguugg gguggacggg gccacagcu 159
<210> 3
   <211> 129
   <212> RNA
   <213> Mus musculus
<400> 3
   aaagggguug aagaauggua uggauucaga augaacuauc agagaaacuc cagagccagc 60
   aggaaacauu auagagccuu ugcuacaaug uccuguuucu uucuuggcuu uuaguucugu 120
   uccacagau 129
<210> 4
   <211> 122
   <212> RNA
   <213> Mus musculus
<400> 4
   gaaagcauuu aauauuuacc aauaguuuau uccgagcuag gguaaagcag ucagugcuag 60
   aaaaaugaga aaacacaaua cauaagaccu cucaagggga gaugcuguua cuguauauac 120
   ug 122
<210> 5
   <211> 147
   <212> DNA
   <213> Drosophila melanogaster
<400> 5
   aaagcgttag atattaaact gtggttgaat tcacaaaata ggccacagtt atgcaataaa        60
   cgctagaaaa aaacggtagt atttaataac gtgttgacta acatctgcgg ataagaagct       120
   ttgcgttttg aggtactaac cacagta                                           147
<210> 6
   <211> 159
   <212> DNA
   <213> Homo sapiens
<400> 6
   gtaagtgtag cctagaaatt ggggctggat ttgaaaatta gccccaattc tgcaattttc        60
   accgcaataa aagcttctcc agttatacat ggtgattggt cttgatgggc tattgtggac       120
   agaggagggt gctaggttgg ggtggacggg gccacagct                              159
<210> 7
   <211> 129
   <212> DNA
   <213> Mus musculus
<400> 7
   aaaggggttg aagaatggta tggattcaga atgaactatc agagaaactc cagagccagc        60
   aggaaacatt atagagcctt tgctacaatg tcctgtttct ttcttggctt ttagttctgt       120
   tccacagat                                                               129
<210> 8
   <211> 122
   <212> DNA
   <213> Mus musculus
<400> 8
   gaaagcattt aatatttacc aatagtttat tccgagctag ggtaaagcag tcagtgctag        60
   aaaaatgaga aaacacaata cataagacct ctcaagggga gatgctgtta ctgtatatac       120
   tg                                                                      122
<210> 9
   <211> 149
   <212> RNA
   <213> Drosophila simulans
<400> 9
   aaagcguuag guauaaaccu gugguugaau ucacaaaaua ggccacaguu augcaauaaa 60
   cgcuagaaaa aaaacgguag uauuuaauaa cguguugacu aacaucugcg gauaaaacag 120
   cuuugcguuu ugagguacua accacagua 149
<210> 10
   <211> 149
   <212> DNA
   <213> Drosophila simulans
<400> 10
   aaagcgttag gtataaacct gtggttgaat tcacaaaata ggccacagtt atgcaataaa 60
   cgctagaaaa aaaacggtag tatttaataa cgtgttgact aacatctgcg gataaaacag 120
   ctttgcgttt tgaggtacta accacagta 149
<210> 11
   <211> 149
   <212> RNA
   <213> Drosophila sechellia
<400> 11
   aaagcguuag guauuaaccu gugguuuaau ucacaaaaua ggccacaguu augcaauaaa 60
   cgcuagaaaa aaaacgguag uauuuaauaa cguguugacu aacaucugcg gauaaaauag 120
   cuuugcguuu ugagguacua accacagua 149
<210> 12
   <211> 149
   <212> DNA
   <213> Drosophila sechellia
<400> 12
   aaagcgttag gtattaacct gtggtttaat tcacaaaata ggccacagtt atgcaataaa 60
   cgctagaaaa aaaacggtag tatttaataa cgtgttgact aacatctgcg gataaaatag 120
   ctttgcgttt tgaggtacta accacagta 149
<210> 13
   <211> 147
   <212> RNA
   <213> Drosophila yakuba
<400> 13
   caagcguugu auaauauuaa acuguggaaa cuucacaaau aggccacagu uaugcaagaa 60
   acgcuagaaa aacuugguag uauuuaauaa cgugcugacu aacgucugcg gauaaaagcu 120
   uugcguuuug agguacuaac cacauua 147
<210> 14
   <211> 147
   <212> DNA
   <213> Drosophila yakuba
<400> 14
   caagcgttgt ataatattaa actgtggaaa cttcacaaat aggccacagt tatgcaagaa 60
   acgctagaaa aacttggtag tatttaataa cgtgctgact aacgtctgcg gataaaagct 120
   ttgcgttttg aggtactaac cacatta 147
<210> 15
   <211> 148
   <212> RNA
   <213> Drosophila erecta
<400> 15
   caugcguuga auaauauuaa acuguggacg aauucccaaa uaagccacag uuaugcaaga 60
   aacgcuagaa aaaauaggua guauuuaaua acgugcugac uaacaucugc ggauaagagc 120
   uuugcguuuu gagguacuaa ccacaaua 148
<210> 16
   <211> 148
   <212> DNA
   <213> Drosophila erecta
<400> 16
   catgcgttga ataatattaa actgtggacg aattcccaaa taagccacag ttatgcaaga 60
   aacgctagaa aaaataggta gtatttaata acgtgctgac taacatctgc ggataagagc 120
   tttgcgtttt gaggtactaa ccacaata 148
<210> 17
   <211> 148
   <212> RNA
   <213> Drosophila ananassae
<400> 17
   uuaggcguuc aaaacauuaa acguuggcug auuuaugauu aggcuagugu uaugcacgca 60
   acgcuagaga aaauugguag uauuuaauaa ugcguuggug gcaaaucuau cgauuuugau 120
   cuucgcauuu ugagguacua gcacaguu 148
<210> 18
   <211> 148
   <212> DNA
   <213> Drosophila ananassae
<400> 18
   ttaggcgttc aaaacattaa acgttggctg atttatgatt aggctagtgt tatgcacgca 60
   acgctagaga aaattggtag tatttaataa tgcgttggtg gcaaatctat cgattttgat 120
   cttcgcattt tgaggtacta gcacagtt 148
<210> 19
   <211> 145
   <212> RNA
   <213> Drosophila pseudoobscura
<400> 19
   cauggcgcuc aaacauuaau cgguggccug auccacacgu cggccacauu uaugcacgca 60
   gcgccagaua auaguaggca gcauuuaaua auguauuggu uccaaaugac ucagacuuuu 120
   gcauuuugag guguuagcca caacg 145
<210> 20
   <211> 145
   <212> DNA
   <213> Drosophila pseudoobscura
<400> 20
   catggcgctc aaacattaat cggtggcctg atccacacgt cggccacatt tatgcacgca 60
   gcgccagata atagtaggca gcatttaata atgtattggt tccaaatgac tcagactttt 120
   gcattttgag gtgttagcca caacg 145
<210> 21
   <211> 145
   <212> RNA
   <213> Drosophila persimilis
<400> 21
   cauggcgcuc aaacauuaau cgguggccug auccacacgu cggccacauu uaugcacgca 60
   gcgccagaua auaguaggca gcauuuaaua auguauuggu uccaaaugac ucagacuuuu 120
   gcauuuugag guguuagcca caaag 145
<210> 22
   <211> 145
   <212> DNA
   <213> Drosophila persimilis
<400> 22
   catggcgctc aaacattaat cggtggcctg atccacacgt cggccacatt tatgcacgca 60
   gcgccagata atagtaggca gcatttaata atgtattggt tccaaatgac tcagactttt 120
   gcattttgag gtgttagcca caaag 145
<210> 23
   <211> 149
   <212> RNA
   <213> Drosophila willistoni
<400> 23
   agaggcguuc aacauuuaac uuuagccguu cuuuuaaacu aagcuauuug uuaugcaagu 60
   aacgcuagaa uauaauuucu gcaacauuua auaaugcuuu ggucguauac uaacaaaaau 120
   cucagcauuu ugaggugucu gcuacaauu 149
<210> 24
   <211> 149
   <212> DNA
   <213> Drosophila willistoni
<400> 24
   agaggcgttc aacatttaac tttagccgtt cttttaaact aagctatttg ttatgcaagt 60
   aacgctagaa tataatttct gcaacattta ataatgcttt ggtcgtatac taacaaaaat 120
   ctcagcattt tgaggtgtct gctacaatt 149
<210> 25
   <211> 141
   <212> RNA
   <213> Drosophila mojavensis
<400> 25
   cccugcguug agcaguaaau ugucgcugau ugauaauuag gcgacgauaa ugcaaacaac 60
   gguagaaaaa acuaacgaca uuuaauaaua cauugaccac uaaaucccau gaacauugca 120
   uuuugaggug ucgcgacaug u 141
<210> 26
   <211> 141
   <212> DNA
   <213> Drosophila mojavensis
<400> 26
   ccctgcgttg agcagtaaat tgtcgctgat tgataattag gcgacgataa tgcaaacaac 60
   ggtagaaaaa actaacgaca tttaataata cattgaccac taaatcccat gaacattgca 120
   ttttgaggtg tcgcgacatg t 141
<210> 27
   <211> 142
   <212> RNA
   <213> Drosophila virilis
<400> 27
   uuaugcguuc aacaguaaau ugucgccgau agauuacuag gcgacaguua ugcaaggcaa 60
   cgcuagaaua agcagacgac auuuaauaau gcauuggccg acaaacucca cggccuuugc 120
   guuuugaggu gucgccacau gu 142
<210> 28
   <211> 142
   <212> DNA
   <213> Drosophila virilis
<400> 28
   ttatgcgttc aacagtaaat tgtcgccgat agattactag gcgacagtta tgcaaggcaa 60
   cgctagaata agcagacgac atttaataat gcattggccg acaaactcca cggcctttgc 120
   gttttgaggt gtcgccacat gt 142
<210> 29
   <211> 141
   <212> RNA
   <213> Drosophila grimshawi
<400> 29
   uugugcguuc gacaguaaau uuucgaugac uguagauagg cgaaaauuau gcaaggcaac 60
   gcuagaccaa uuagaugacg acauuuaaua augcauuggc cgauuaacuc agaucuugca 120
   uuuugaggug ucgccacaug u 141
<210> 30
   <211> 141
   <212> DNA
   <213> Drosophila grimshawi
<400> 30
   ttgtgcgttc gacagtaaat tttcgatgac tgtagatagg cgaaaattat gcaaggcaac 60
   gctagaccaa ttagatgacg acatttaata atgcattggc cgattaactc agatcttgca 120
   ttttgaggtg tcgccacatg t 141
<210> 31
   <211> 17
   <212> DNA
   <213> artificial sequence
<220>
   <223> séquence cible au sein du gène Ir56d
<400> 31
   tggttgaatt cacaaaa 17
<210> 32
   <211> 16
   <212> DNA
   <213> artificial sequence
<220>
   <223> séquence cible au sein du gène buttonhead
<400> 32
   ttgaattcac aaaata 16
<210> 33
   <211> 16
   <212> DNA
   <213> artificial sequence
<220>
   <223> séquence cible au sein du gène klarsicht
<400> 33
   aattcacaaa ataggc 16
<210> 34
   <211> 16
   <212> DNA
   <213> artificial sequence
<220>
   <223> séquence cible au sein du gène CG3262
<400> 34
   aagcgttaga tattaa 16
<210> 35
   <211> 16
   <212> DNA
   <213> artificial sequence
<220>
   <223> séquence cible au sein des gènes CG30502 et CG11125
<400> 35
   acatctgcgg ataaga 16
<210> 36
   <211> 16
   <212> DNA
   <213> artificial sequence
<220>
   <223> séquence cible au sein du gène CG9339
<400> 36
   aagctttgcg ttttga 16
<210> 37
   <211> 16
   <212> DNA
   <213> artificial sequence
<220>
   <223> séquence cible au sein du gène CG40006
<400> 37
   agaagctttg cgtttt 16

## Description

La présente invention concerne l'utilisation de séquences ARN particulières en tant que médicament. Elle concerne plus précisément l'utilisation de petits ARNs nucléolaires (snoARNs) dont les inventeurs ont démontré l'implication dans les mécanismes du vieillissement. Les snoARNs de l'invention peuvent être utilisés en particulier pour augmenter la résistance au stress d'un sujet, pour lutter contre les effets délétères du vieillissement, typiquement pour prévenir ou traiter une maladie dégénérative, en particulier neurodégénérative, une laminopathie, en particulier le syndrome de Hutchinson-Gilford (HGPS) (connu aussi sous le nom de progéria), le diabète, l'obésité ou un cancer ainsi que, plus généralement, pour prolonger la durée de vie d'un sujet. Les snoARNs de l'invention peuvent également être utilisés dans le traitement de l'infertilité.

Sont également décrits les vecteurs, cellules, animaux transgéniques, compositions capables d'exprimer un snoARN selon l'invention et kits comprenant les acides nucléiques, vecteurs, cassettes et/ou cellules décrits dans le présent texte, ainsi que les méthodes utilisant l'un des produits précités de l'invention comme outil d'identification d'une molécule active dans la prévention ou le traitement d'une pathologie, d'une anomalie, d'un désordre ou d'une détérioration apparente ou fonctionnelle lié(e) à un mécanisme du vieillissement.

### ARRIERE PLAN TECHNOLOGIQUE

Les mécanismes du vieillissement (c'est-à-dire le déclin progressif de la capacité à survivre et à se reproduire avec l'âge) ont laissé la société et la communauté scientifique perplexe pendant des siècles. Les deux théories qui prévalent à l'heure actuelle soutiennent pour l'une que le vieillissement résulterait d'une voie évolutive génétiquement préprogrammée, et pour l'autre, que le vieillissement serait une conséquence normale de l'existence au cours de laquelle s'accumulent les dommages cellulaires et moléculaires. Ces dommages incluraient des dommages oxydatifs provoqués par les radicaux libres, des mitochondries défectueuses, des mutations somatiques, le raccourcissement progressif des télomères, la mort cellulaire programmée et la prolifération des cellules détériorées, etc. (Semsei I. (2000) On the nature of aging. Mech Aging Dev 117:93-108).

Il a été montré, sur des organismes tels que la levure et la souris, que la réduction de l'apport calorique exerce un impact positif décisif sur l'allongement de la durée de vie (Sohal, R S, Weindruch, R (1998) Oxidative stress, caloric restriction, and aging. Science 273:59-63 ; Finch, C E, Revkun, G. (2001) The genetics of aging. Annu. Rev. Genom. Hum. Genet. 2:435-462). Des études récentes ont mis en évidence que la restriction calorique serait également efficace sur les primates, dont les humains (Roth, G S, Lasnikov, V, Lesnikov, M, Ingram, D K, Land, M A (2001) Dietary caloric restriction prevents the age-related decline in plasma melatonin levels of rhesus monkeys. J Clin Endocrinol Metab. 86:3292-5 ; Roth G S, Lane M A, Ingramn D K, Mattison J A, Elahi D, Tobin J D, Muller D, Metter E J (2002) Biomarkers of caloric restriction may predict longevity in humans. Science. 297:811-813 ; Walford R L, Mock D, Verdery R, MacCallum T. (2002) Calorie restriction in biosphere 2: alterations in physiologic, hematologic, hormonal, and biochemical parameters in humane restricted for a 2-year period. J Gerontol A Biol Sci Med Sci 57:211-24). Malheureusement, il est probable que la plupart des humains ne soient pas capable de suivre le régime diététique strict requis pour bénéficier de cette découverte.

De nombreux groupes de recherche se consacrent depuis quelques années à l'identification des gènes et des voies de signalisation impliqués dans le processus du vieillissement. Les études qui s'y rapportent sont réalisées sur de nombreux organismes dont la levure *Saccharomyces cerevisiae*, le ver *Caenorhabditis elegans* et la drosophile *Drosophila melanogaster* (Fontana et al., 2010), et ont permis l'identification d'une liste grandissante de gènes. Nombre d'entre eux sont impliqués dans la signalisation hormonale et sont conservés au sein d'une grande variété d'organismes eucaryotes.

Il est devenu clair, tout du moins pour les espèces inférieures, que les voies responsables du développement et de la croissance au début de la vie conservent une influence tout au long de la vie et conditionnent même en partie sa durée. Ces études ont par ailleurs indirectement mis en évidence l'importance de la capacité métabolique et de la résistance au stress pour évaluer la durée de vie d'un sujet.

Par exemple, les mutants du gène clk-1 de *Caenorhabditis elegans* ont été impliqués dans la diminution de la production extra-mitochondriale d'espèces réactives de l'oxygène (Hekimi, S, Guarente, L. (2003) Genetics and the specificity of the aging process. Science 299:1351-1354). La demande de brevet WO 98/17823 décrit la fonction du gène clk-1 dans le processus de développement et la longévité. Elle revendique notamment une méthode pour augmenter la durée de vie d'un individu par la régulation de l'expression du gène clk-1.

Une mutation du gène Methuselah (qui code pour un récepteur couplé aux protéines G) a par ailleurs été décrite comme capable d'augmenter d'environ 35% la durée de vie de la Drosophile (US 6,303,768).

Le raccourcissement des télomères est par ailleurs connu comme un mécanisme responsable du vieillissement cellulaire. Chez les vertébrés, la télomérase est une transcriptase inverse de type RiboNucleoProtein (RNP) dont le rôle est de maintenir la longueur des télomères par addition de l'ADN télomérique au bout des chromosomes dans les cellules eucaryotes en division (Kiss, 2002).

Chez l'humain, une mutation dans la boîte H/ACA du snoARN de la télomérase engendre une maladie génétique pléiotropique, la dyskératose congénitale, dont les patients présentent des télomères plus courts (Mitchell et al., 1999; Vulliamy et al., 2001). Malgré les efforts réalisés par la communauté scientifique pour décrypter les mécanismes du vieillissement et identifier de nouvelles cibles thérapeutiques permettant de retarder ce processus et/ou de combattre ses effets délétères, les outils disponibles demeurent insuffisants. Le besoin d'outils permettant de « vieillir bien », c'est-à-dire sans les maladies et désagréments généralement associés à la vieillesse, se fait d'autant plus sentir que les progrès de la médecine associés à l'amélioration générale des conditions de vie ont déjà permis à la population humaine, en seulement quelques dizaines d'années, d'augmenter son espérance de vie de manière tout à fait significative.

### RESUME DE L'INVENTION

La présente invention concerne l'utilisation dans un contexte prophylactique ou thérapeutique de séquences ARN d'intérêt dont les inventeurs ont démontré l'implication particulière dans les mécanismes du vieillissement. Ils ont en particulier démontré la capacité de ces séquences à augmenter de manière spectaculaire la durée de vie d'un sujet en enrayant les mécanismes du vieillissement et en luttant contre les effets délétères associés. Les séquences de l'invention permettent ainsi de lutter contre les maladies associées à la vieillesse telles que les maladies dégénératives, les laminopathies et les cancers, mais également contre le diabète et l'obésité. Elles se sont par ailleurs révélées efficaces dans le traitement des problèmes d'infertilité ou de stérilité (i.e., difficulté à, ou impossibilité de, donner la vie) ainsi que dans l'augmentation de la résistance des sujets, en particulier des sujets ayant atteint leur maturité sexuelles, de préférence des sujets âgés, au stress.

Un premier objet de l'invention concerne une séquence d'ARN isolée ou synthétique comprenant la séquence SEQ ID NO : 1, une séquence homologue, de préférence une séquence orthologue, ou une séquence analogue sur le plan fonctionnel, pour une utilisation en tant que médicament.
Les inventeurs ont démontré qu'à la séquence ARN SEQ ID NO : 1 découverte chez la drosophile correspondent des séquences orthologues chez les mammifères tels que les primates et l'être humain, et chez les rongeurs tels que la souris et le rat. L'invention couvre ainsi également l'utilisation, dans l'une ou l'autre des applications décrites dans le présent texte, d'une séquence d'ARN isolée ou synthétique choisie de préférence parmi la séquence SEQ ID NO : 2 d'origine humaine et l'une des séquences SEQ ID NO : 3 et SEQ ID NO : 4 d'origine murine. Les séquences SEQ ID NO : 2, 3, 4, 9, 11, 13, 15, 17, 19, 21, 22, 24, 26, et 28 identifiées dans le tableau n°1 du présent texte sont des exemples de séquences orthologues de la séquence SEQ ID NO : 1.
Un autre objet de l'invention est une séquence ADN codant une séquence ARN d'intérêt selon l'invention.

L'invention concerne aussi un vecteur permettant l'expression *in vitro*, *ex vivo* ou *in vivo*, d'une séquence ARN selon l'invention.
L'invention concerne également une cellule comprenant une séquence ARN selon l'invention ou transformée à l'aide d'un vecteur selon l'invention, ainsi qu'une composition comprenant un produit selon l'invention et un support acceptable sur le plan diététique ou pharmaceutique.
L'invention concerne par ailleurs l'utilisation d'une séquence ARN, d'un vecteur, d'une cellule ou d'une composition selon l'invention, de manière continue ou séquentielle, pour restaurer ou moduler l'expression de ladite séquence ARN *in vivo*, *in vitro* ou *ex vivo.*
Un autre objet de l'invention est une souris transgénique dont le génome a été modifié génétiquement pour prévenir ou modifier, de préférence altérer, l'expression d'une séquence ARN d'intérêt selon l'invention, typiquement de l'une et/ou l'autre des séquences SEQ ID NO : 3 et SEQ ID NO : 4.

### DESCRIPTION DETAILLEE

L'invention résulte de la mise en évidence par les inventeurs de l'influence d'une petite séquence d'ARN nucléolaire (snoARN:Ψ28S-1153 nommé « *jouvence* » identifié dans le présent texte en tant que SEQ ID NO :1) sur la durée de vie des drosophiles. Ce snoARN a été identifié dans le cadre d'un criblage systématique des snoARNs associés au génome de la drosophile (Huang et al., 2005) sans qu'aucune fonction ne lui soit associé jusqu'à ce jour.
Les inventeurs ont caractérisé cette séquence et ont notamment montré qu'une délétion génomique de 632 paires de bases de la région F4 du génome de la drosophile, contenant la séquence du snoARN:Ψ28S-1153, raccourcie la durée de vie des drosophiles d'environ 30%. Ils ont ensuite mis en évidence que la surexpression de cette séquence ARN, à l'aide par exemple d'un transgène contenant l'ADN génomique codant ce snoARN, augmente de manière spectaculaire la durée de vie des drosophiles d'environ 100%. Les mouches surexprimant la séquence ARN de l'invention vivent ainsi jusqu'à deux fois plus longtemps. Les inventeurs ont par ailleurs identifié et détecté l'expression de séquences « *jouvence* » orthologues chez la souris et chez l'être humain.

### Caractéristiques des séquences ARNs et autres produits de l'invention

Comme expliqué précédemment, l'invention concerne une séquence ribonucléotidique (séquence ARN), isolée ou synthétique, présentant les caractéristiques fonctionnelles de la séquence SEQ ID NO : 1 identifiée à l'origine chez la drosophile (*Drosophila melanogaster*), pour une utilisation en tant que médicament chez un sujet susceptible d'en bénéficier, typiquement chez un animal ou un insecte, par exemple un mammifère ou un rongeur, de préférence chez un être humain.

Par « *médicament* », on entend une substance possédant des propriétés préventives ou curatives. Dans le cadre de l'invention, un médicament est destiné à guérir, favoriser la guérison, soulager ou prévenir, chez un sujet tel que défini précédemment, une pathologie, anomalie ou altération liée à un processus de vieillissement anormal ou simplement indésirable.

Chez la drosophile, la séquence ARN d'intérêt est comprise dans la région génomique identifiée en tant que région F4, elle-même située sur le chromosome 2R. Cette séquence nucléotidique est, pour la première espèce de drosophile (*Drosophila melanogaster*) étudiée par les inventeurs, constituée de 148 paires de bases. Elle est identifiée dans le présent texte en tant que SEQ ID NO : 1.

Les homologues/orthologues, ainsi que les analogues et variants fonctionnels de la séquence SEQ ID NO : 1 sont, au même titre que cette dernière, des séquences d'intérêt objets de la présente invention.
Le terme « *homologue* » est utilisé dans le contexte de la présente invention pour désigner une structure dont la séquence nucléotidique est identique ou suffisamment proche de celle de la séquence SEQ ID NO : 1, identifiée chez cette première espèce de drosophile, pour être considérée comme son équivalent au sein d'une autre espèce (de drosophile, d'insecte, ou de tout autre animal). Une séquence est considérée comme homologue de la séquence SEQ ID NO : 1 si elle présente avec cette dernière, ou avec l'un de ses fragments d'au moins 50 nucléotides consécutifs, une identité de séquence d'au moins 70%, 80% ou 85%, de préférence d'au moins 90 ou 95%, encore plus préférentiellement d'au moins 96, 97, 98 ou 99% obtenus par exemple par le programme d'alignement de séquences blastN (Altschul *et al.*, 1990), ou encore par le programme INFERNAL pour "INFERence of RNA ALignment", qui identifie des séquences d'ADN à partir de la structure des ARNs et de leur similarité de séquence. Deux séquences génétiques homologues de deux espèces différentes sont considérées comme orthologues si elles descendent d'une séquence unique présente dans le dernier ancêtre commun aux deux espèces.

Le pourcentage d'identité est déterminé en comparant deux séquences dont un alignement optimal a été réalisé. Le pourcentage d'identité est calculé en déterminant le nombre de positions pour lesquelles un résidu identique apparaît sur les deux séquences à la même position divisé par le nombre de positions totales et multiplié par 100. L'alignement optimal des deux séquences peut être obtenu par exemple avec un algorithme de recherche d'homologie local (Smith & Watherman, 1981) ou des systèmes équivalents connus de l'homme du métier.

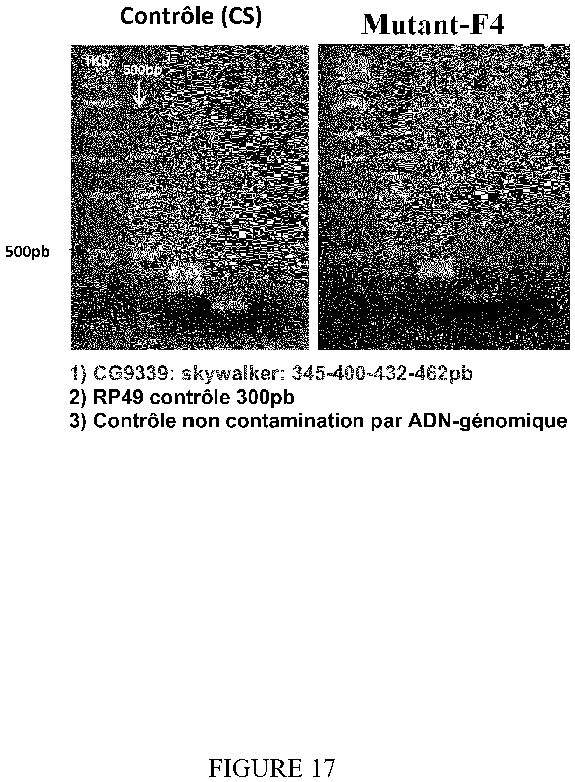

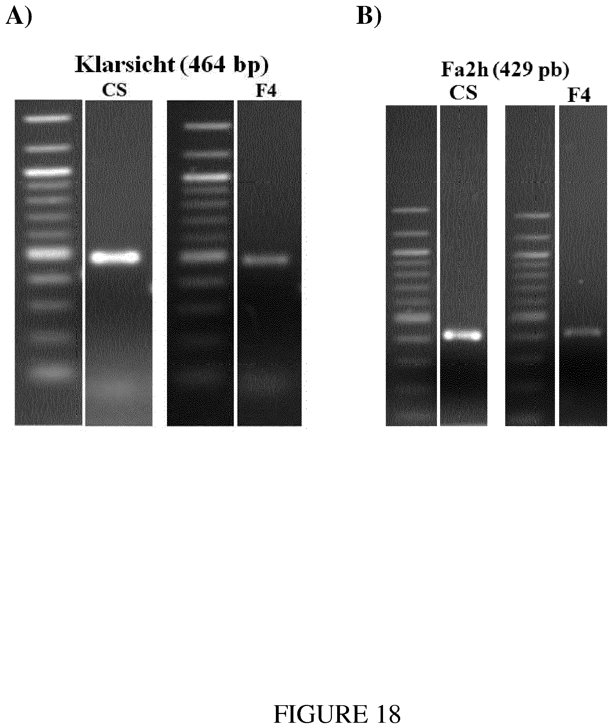

Le terme « *analogue* » désigne tout mimétique qui est un composé chimique existant dans la nature et isolé à partir de cette dernière, ou fabriqué artificiellement, et qui possède au moins une des fonctions endogènes de la séquence nucléotidique qu'il imite (séquence ARN équivalente sur le plan fonctionnel), par exemple l'ensemble des fonctions endogènes de la séquence nucléotidique qu'il imite. Un exemple de composé analogue consiste par exemple en une séquence permettant l'épissage des mêmes gènes. Un objet particulier de l'invention concerne ainsi une séquence analogue de SEQ ID NO :1 qui permet l'épissage d'un ou de plusieurs gènes choisis parmi les gènes Ir56d, buttonhead, klarsicht, CG3262, CG30502 (« fatty acid 2-hydroylase »- *fa2h*), CG11125, CG9339, et CG40006 de *Drosophila melanogaster.* Des expériences réalisées par les inventeurs ont démontré par exemple que le gène CG9339 (appelé : *skywalker*) (Uytterhoeven *et al.*, 2011) n'est pas épissé correctement dans le mutant F4, indiquant que le snoARN *jouvence* est impliqué dans l'épissage alternatif de ce gène (cf. Figure 17). Des résultats similaires ont également été obtenus pour un transcrit du gène *klarsicht,* pour lequel on observe moins d'ARN épissé chez le mutant F4 que chez les mouches contrôles de type sauvage Canton-S (Figure 18A), ainsi que pour le gène CG30502, codant pour le gène *fa2h* (Figure 18B), ce dernier ayant été impliqué chez l'humain dans diverses formes de démyélinisation, telle que les neuro-dégénérescences lié à l'accumulation de fer, des leucodystrophies (typiquement celles liées aux mutations du gène fa2h), et une paraplégie spastique héréditaire (typiquement la paraplégie spastique héréditaire SPG35) (Dick et al., 2008; 2010 ; Pierson et al., 2012).

Le terme *"variant fonctionnel"* désigne tout acide nucléique présentant une ou plusieurs modifications ou mutation (c'est-à-dire par exemple une délétion, une substitution ou une addition d'une ou plusieurs bases) par rapport aux séquences parentes décrites dans la présente demande, et permettant l'une des applications décrites dans le cadre de la présente invention.
Une séquence considérée, au sens de l'invention, comme l'homologue, de préférence l'orthologue, d'une séquence de référence est un exemple de variant fonctionnel au sens de l'invention de ladite séquence de référence. Une telle séquence peut être naturelle, recombinante ou synthétique.

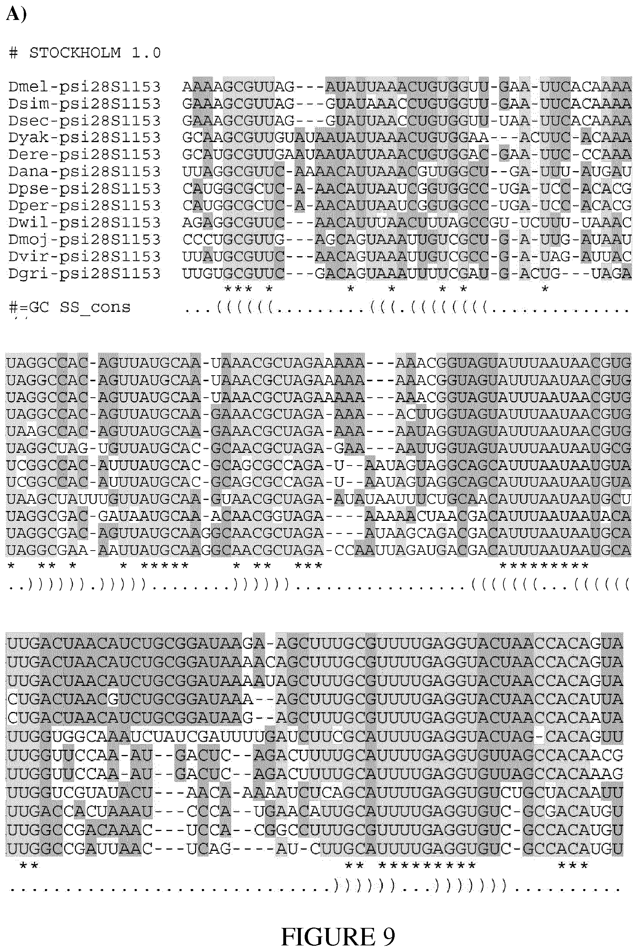

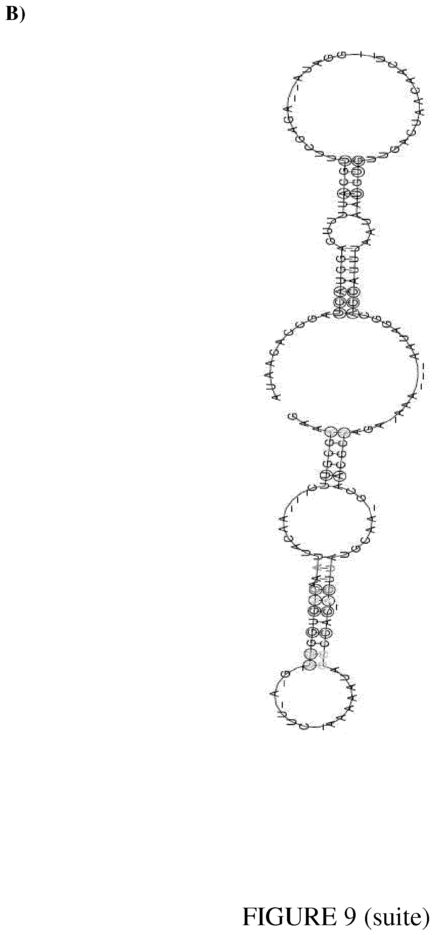

La séquence SEQ ID NO : 1 est une séquence très conservée au cours de l'évolution et présente chez nombre d'espèces animales. Les inventeurs ont ainsi notamment montré que cette séquence est présente chez les 12 autres espèces de drosophiles dont le génome est connu. Des exemples de séquences homologues/orthologues à la séquence ARN SEQ ID NO :1 comprennent ainsi les séquences ARN SEQ ID NO : 9, 11, 13, 15, 17, 19, 21, 22, 24, 26, et 28 identifiées dans le tableau n°1 du présent texte (cf. Figure 9).

Les inventeurs ont par ailleurs identifié une séquence ARN orthologue chez l'être humain, localisée sur le chromosome 11, en position 12822722-12822811. Il s'agit de la séquence identifiée dans le présent texte en tant que SEQ ID NO : 2. Les inventeurs ont aussi identifié deux séquences ARN orthologues chez la souris (il s'agit des séquences identifiées respectivement en tant que SEQ ID NO : 3 et 4 dans le présente texte), localisées sur le chromosome 15, en position 30336889-30337017 (SEQ ID NO : 7) et sur le chromosome 18, en position 6495012-6495091 (SEQ ID NO : 8).
Des séquences nucléotidiques d'intérêt identifiées chez la drosophile, l'homme et la souris sont listées dans le tableau 1 ci-dessous.

**Tableau n°1 :**

| Espèce | Séquences ARN (de 5' vers 3') | SEQ ID NO: |
|---|---|---|
| *Drosophila melanogaster* | | 1 |
| Homme | | 2 |
| Souris-1 | | 3 |
| Souris-2 | | 4 |
| *Drosophila simulans* | | 9 |
| *Drosophila sechellia* | | 11 |
| *Drosophila yakuba* | | 13 |
| *Drosophila erecta* | | 15 |
| *Drosophila ananassae* | | 17 |
| *Drosophila pseudoobscura* | | 19 |
| *Drosophila persimilis* | | 21 |
| *Drosophila willistoni* | | 22 |
| *Drosophila mojavensis* | | 24 |
| *Drosophila virilis* | | 26 |
| *Drosophila grimshawi* | | 28 |

| | **Séquences ADN correspondantes** | |
|---|---|---|
| *Drosophila melanogaster* | | 5 |
| humain | | 6 |
| souris-1 | | 7 |
| Souris-2 | | 8 |
| *Drosophila simulans* | | 10 |
| *Drosophila sechellia* | | 12 |
| *Drosophila yakuba* | | 14 |
| *Drosophila erecta* | | 16 |
| *Drosophila ananassae* | | 18 |
| *Drosophila pseudoobscura* | | 20 |
| *Drosophila persimilis* | | 22 |
| *Drosophila willistoni* | | 24 |
| *Drosophila mojavensis* | | 26 |
| *Drosophila virilis* | | 28 |
| *Drosophila grimshawi* | | 30 |

Un objet particulier de l'invention concerne l'utilisation de la séquence ARN SEQ ID NO :1 pour identifier un gène cible, caractérisée en ce que ladite séquence SEQ ID NO :1 est capable de reconnaître une séquence cible au sein d'un gène de *Drosophila melanogaster* choisi de préférence parmi Ir56d, buttonhead, klarsicht, CG3262, CG30502 (*fatty acid 2-hydroylase* : *fa2h*) et CG11125, CG9339, CG40006, ladite séquence cible étant choisie de préférence parmi SEQ ID NO :31 (TGGTTGAATTCACAAAA), SEQ ID NO :32 (TTGAATTCACAAAATA), SEQ ID NO :33 (AATTCACAAAATAGGC), SEQ ID NO :34 (AAGCGTTAGATATTAA), SEQ ID NO :35 (ACATCTGCGGATAAGA), SEQ ID NO :36 (AAGCTTTGCGTTTTGA), et SEQ ID NO :37 (AGAAGCTTTGCGTTTT), ou une séquence analogue de celle-ci.

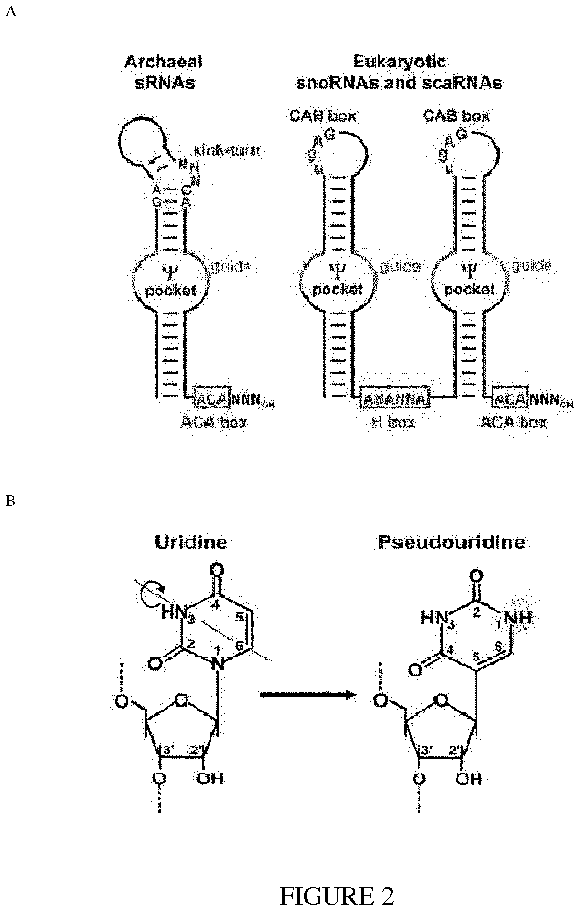

Dans le cadre de la présente invention, les séquences ARN d'intérêt se présentent de préférence sous la forme de snoARNs. Dans le contexte de l'invention, le terme « *snoARNs* » englobe un groupe d'ARNs non-codants présent dans toutes les cellules eucaryotes. Ils sont localisés dans le noyau et plus particulièrement dans le nucléole où ils sont associés à des protéines avec lesquelles ils forment les petites ribonucléoprotéines nucléolaires (small nucleolar ribonucleoproteins ou snoRNPs). Ils sont généralement produits à partir des introns des pré-ARNms. Au niveau évolutif, ces ARNs non-codants sont présents des archaebactéries jusqu'aux mammifères. Les snoARNs peuvent avoir plusieurs fonctions telles que la modification des ARN ribosomaux, comme la 2'-O-ribose méthylation ou la pseudo-uridilation des différentes classes d'ARN. Ils ont été impliqués dans le processus nucléolytique des ARNs ribosomaux ou encore dans la synthèse de l'ADN télomérique (Kiss, 2002 ; Kiss et al., 2010 ; Ye, 2007). On distingue deux classes principales de snoARNs : celle comprenant une boîte (Box) C/D et celle comprenant une boîte H/ACA (Figure 2A). Les boîtes C/D servent de guide pour la 2'-O-ribose méthylation sur des sites spécifiques, alors que les boîtes H/ACA dirigent la conversion de l'uridine en pseudo-uridine (Kiss, 2002 ; Gardner et al., 2010 ; Huang et al., 2005) (Figure 2B).
Le snoARN:Ψ28S-1153 (*jouvence*) d'intérêt, identifié chez une première espèce de drosophile, appartient à la classe H/ACA, c'est-à-dire que sa structure comprend une boîte H/ACA. Il serait donc impliqué dans la pseudo-uridinylation (c'est-à-dire la conversion de l'uridine en pseudo-uridine) lors de la maturation des ARN ribosomaux, et régulerait la synthèse protéique de certains gènes.

Les snoARNs selon l'invention peuvent être chimiquement modifiés, typiquement pour augmenter la résistance des snoARNs aux nucléases, pour conférer une meilleure affinité/sélectivité et donc une meilleure fonctionnalité aux snoARNs. Les modifications concernent typiquement un nucléoside ou un lien internucléosidique. Elles peuvent par exemple concerner le sucre (typiquement la position 2' du sucre), la base azotée du nucléoside (qui peut typiquement comprendre un substituant en position 2, 4 ou 6) ou un (ou plusieurs) groupement(s) phosphate.
La modification peut consister par exemple en une amination, une halogénation par exemple fluoration, ou en une alkylation par exemple méthylation. Un groupement sucre modifié peut ainsi être choisi par exemple parmi les groupes 2'-O-méthyle et 2'-O-méthoxyéthyl.
Un nucléotide modifié peut être un nucléotide comprenant une base hétérocyclique incapable de créer des liaisons hydrogènes avec les bases hétérocycliques de l'ADN ou de l'ARN.
La modification du lien internucléosidique est par exemple choisie parmi une liaison phosphorothioate, méthylphosphonate, phosphotriester, phosphorodithioate et phosphoselenate.

La présente invention concerne également les séquences d'acide désoxyribonucléique (ADN) responsables de l'expression des séquences ARN d'intérêt de l'invention. La séquence ADN permettant l'expression de la séquence ARN SEQ ID N : 1 est la séquence identifiée dans la présente invention en tant que SEQ ID NO : 5, celle permettant l'expression de la séquence SEQ ID N : 2 est la séquence identifiée dans la présente invention en tant que SEQ ID NO : 6, celle permettant l'expression de la séquence SEQ ID N : 3 est la séquence identifiée dans la présente invention en tant que SEQ ID NO : 7, et celle permettant l'expression de la séquence SEQ ID N : 4 est la séquence identifiée dans la présente invention en tant que SEQ ID NO : 8.

Un objet de l'invention concerne ainsi une séquence d'ADN isolée ou synthétique sélectionnée dans le groupe comprenant les séquences SEQ ID NO : 5, SEQ ID NO : 6, SEQ ID NO : 7, SEQ ID NO : 8, SEQ ID NO : 10, SEQ ID NO : 12, SEQ ID NO : 14, SEQ ID NO : 16, SEQ ID NO : 18, SEQ ID NO : 20, SEQ ID NO : 22, SEQ ID NO : 24, SEQ ID NO : 26, SEQ ID NO : 28, et SEQ ID NO : 30, et caractérisée en ce qu'elle permet l'expression d'une séquence ARN d'intérêt selon l'invention.

Tout comme les snoARNs, les ADNs selon l'invention peuvent être modifiés chimiquement (voir ci-dessus les modification chimiques possibles).

L'invention concerne également toute cassette d'expression recombinante, caractérisée en ce qu'elle comprend une séquence d'acide nucléique d'intérêt selon l'invention telle que définie dans le présent texte. Le terme cassette d'expression désigne une construction d'acide nucléique comprenant une séquence d'acide nucléique permettant l'expression d'une séquence ARN d'intérêt selon l'invention et une région régulatrice, liées de manière opérationnelle. L'expression "lié de manière opérationnelle" indique que les éléments sont combinés de manière à ce que l'expression de la séquence d'acide nucléique (responsable de l'expression de la séquence ARN d'intérêt) et/ou le ciblage de la séquence ARN d'intérêt soient sous contrôle du promoteur transcriptionnel. Typiquement, la séquence du promoteur est placée en amont de la séquence d'acide nucléique, à une distance de celle-ci compatible avec le contrôle de l'expression. Des séquences d'espacement peuvent être présentes, entre les éléments régulateurs et la séquence codante, dès lors qu'elles n'empêchent pas l'expression et/ou le ciblage de la séquence ARN d'intérêt.
Un autre objet de l'invention concerne tout vecteur (d'expression) comprenant un acide nucléique ou une cassette tels que définis précédemment permettant l'expression d'une séquence ARN d'intérêt selon l'invention dans une cellule hôte ou dans un organisme hôte. Le vecteur peut être un ADN ou un ARN, circulaire ou non, simple- ou double-brin. Il s'agit typiquement d'un plasmide, d'un phage, d'un vecteur viral (choisi par exemple parmi un vecteur adénoviral, un vecteur rétroviral, un vecteur viral adéno-associé, un vecteur lentiviral, un vecteur poxvirus, un vecteur herpétique, etc.), d'un cosmide, ou d'un chromosome artificiel. Il s'agit avantageusement d'un vecteur capable de transformer une cellule eucaryote, de préférence une cellule animale, typiquement une cellule humaine, de préférence une cellule d'origine intestinale ou ovarienne. De tels vecteurs sont bien connus de l'homme du métier et ont notamment été décrits dans la demande de brevet WO 06/085016 ou dans les articles de Barton and Medzhitov, 2002 ; Tiscornia et al., 2004 ; Xia et al., 2002 et Shen et al., 2003.
Un vecteur préféré permettant l'expression d'une séquence ARN d'intérêt selon l'invention peut être choisi parmi par exemple un plasmide, un cosmide, un vecteur viral ou un phage. Un vecteur préféré utilisable en particulier chez la Drosophile est le vecteur pChs-Gal4, dans lequel on insère, en amont du facteur de transcription Gal4, une séquence ADN spécifique, connue pour réguler l'expression d'un gène donné dans un tissue donné, telle que par exemple Myo1A-Gal4, escargot-Gal4, Su(H)-GBE-Gal4, Delta-Gal4. En parallèle, un deuxième vecteur (pUAS-snoARN) comprenant le gène effecteur d'intérêt, ici le snoARN, est de préférence placé en aval des séquences régulatrices UAS (Upstream Activating Sequence), ces dernières étant reconnues par le facteur de transcription P[GAL4]. Chacun de ces deux vecteurs est ensuite introduit par transgenèse dans un animal. Ces deux lignées animales sont ensuite croisées. Ce système est dit : système binaire d'expression P[GAL4] (Brand and Perrimon, 1993; Elliott and Brand, 2008). Ainsi, plus spécifiquement, les vecteurs Myo1A-Gal4, escargot-Gal4, Su(H)-GBE-Gal4, Delta-Gal4 comportent un promoteur et des éléments régulateurs favorisant l'expression de ladite séquence ARN dans les cellules intestinales, alors que les vecteurs nanos-Gal4, ou MTD-(maternal-Tubulin)-Gal4 comportent un promoteur et des éléments régulateurs favorisant l'expression de ladite séquence ARN dans les ovaires.

Les vecteurs de l'invention peuvent comprendre, en outre, une origine de réplication, un gène de sélection, un gène rapporteur et/ou une séquence de recombinaison, etc. Les vecteurs peuvent être construits par des techniques classiques de biologie moléculaire, bien connues de l'homme du métier, utilisant par exemple des enzymes de restriction, de ligature, des clonages, réplication, etc. Des exemples spécifiques de vecteurs au sens de l'invention sont fournis dans la partie expérimentale. Ces vecteurs peuvent inclure par exemple le système binaire P[GAL4] (susmentionné) (Brand and Perrimon, 1993; Elliott and Brand, 2008).
L'invention concerne également une cellule comprenant une séquence ARN selon l'invention ou transformée à l'aide d'une construction ou d'un vecteur selon l'invention. La cellule est de préférence une cellule intestinale ou ovarienne. De manière encore plus préférée, il s'agit d'une cellule souche intestinale, d'un entéroblaste, d'un entérocyte, d'une cellule entéro-endocrine, d'une cellule nourricière, ou d'un ovocyte.

L'invention comprend également l'utilisation d'un produit selon l'invention, typiquement d'une séquence ARN, d'une séquence ADN, d'un vecteur ou d'une cellule selon l'invention, de manière continue ou séquentielle, pour restaurer ou moduler, typiquement amplifier, *in vitro, ex vivo* ou *in vivo*, l'expression de la séquence ARN d'intérêt selon l'invention.

L'invention concerne par ailleurs une souris transgénique dont le génome (typiquement la séquence SEQ ID NO :7 ou SEQ ID NO :8) a été modifié génétiquement, à l'aide de techniques bien connues de l'homme du métier, pour prévenir ou modifier, par exemple augmenter ou diminuer l'expression, de préférence altérer l'expression, de l'une ou l'autre des séquences SEQ ID NO : 3 et SEQ ID NO :4.
La souris est avantageusement modifiée génétiquement par recombinaison homologue (knock-in, knock-out, knock-down) à l'aide de techniques connues de l'homme du métier, incluant par exemple l'utilisation du système CRE/LOX.

L'invention concerne également les compositions comprenant une séquence ARN, une séquence ADN, une construction, un vecteur ou une cellule selon l'invention.
Les séquences ARN de l'invention sont très stables et très résistantes *in vitro.* Toutefois, dans le cas d'une administration à un sujet à traiter, les compositions selon l'invention peuvent en outre comprendre de manière avantageuse un support acceptable sur le plan diététique ou pharmaceutique.
L'expression « *support acceptable sur le plan diététique* » désigne un véhicule permettant au sujet d'ingérer et de digérer sans risque la composition comprenant une séquence ARN, une construction, un vecteur ou une cellule selon l'invention comprenant une telle séquence, et capable de protéger ladite séquence de toute agression, en particulier liée à la digestion de l'aliment, susceptible de l'altérer avant qu'elle n'ait produit son action thérapeutique.
L'expression « *support acceptable sur le plan pharmaceutique* » désigne un véhicule permettant l'administration sans risque de la composition comprenant une séquence ARN, une construction, un vecteur ou une cellule selon l'invention comprenant une telle séquence, selon l'une des voies d'administration possibles décrites ci-dessous.
De manière avantageuse, le véhicule de la présente composition facilite la pénétration de l'ARN d'intérêt, de la construction ou du vecteur d'expression selon l'invention dans les cellules, idéalement dans des cellules particulières telles qu'identifiées dans le présent texte, du sujet à traiter, et/ou protège l'ARN, la construction ou le vecteur d'expression d'une éventuelle dégradation pouvant nuire à son efficacité.
Le choix du véhicule ainsi que la teneur en substance active dans le véhicule sont généralement déterminés par rapport à la solubilité et aux propriétés chimiques de la substance active, au mode d'administration et aux caractéristiques du sujet à traiter.
Parmi les véhicules ou supports utilisables, acceptables sur le plan pharmaceutique, on peut notamment citer les polymères cationiques naturels tels que le chitosan ou l'atelocollagène, ou synthétiques tels que le poly(L-lysine), le polyéthylènimine (PEI) ou les dendrimères, qui forment des complexes avec les acides nucléiques de l'invention ; les liposomes ; les liposomes cationiques ; les liposome galactosylés ; les liposomes recouverts d'un ligand leur permettant de cibler un type de cellules tels que les immunoliposomes recouverts d'un anticorps spécifique de la cellule cible (Zheng et al., 2009) ; les liposomes disposés au sein d'une nanoparticule formée par des polymères (Carmona et al., 2009) ou encore des films multicouches de polycations et polyanions.
Les excipients tels que le lactose, le citrate de sodium, le carbonate de calcium, le phosphate de dicalcium et les agents de désintégration tels que l'amidon, les acides alginiques et certains silicates complexes combinés avec des lubrifiants tels que le stéarate de magnésium, le sodium lauryl sulfate et le talc peuvent être utilisés pour préparer des comprimés. Pour préparer une pastille, il est avantageux d'utiliser du lactose et des polyéthylènes glycols à fort poids moléculaire. Les suspensions aqueuses contiennent des agents émulsifiants ou des agents facilitant la suspension. Les diluants tels que le sucrose, l'éthanol, le polyéthylène glycol, le propylène glycol, le glycérol et le chloroforme ou des mélanges de ceux-ci sont également utilisables.
Des adjuvants tels que des sels d'aluminium (par exemple de l'hydroxyde d'aluminium, du phosphate d'aluminium, du sulfate d'aluminium), des substances tensio-actives (telles que lysilécithine, polyols pluroniques, polyanions, peptides et émulsions), l'adjuvant de Freund complet et incomplet, l'adjuvant MPL-TDM (Lipide monophosporyle A, dicorynomycoiate tréhalose synthétique), de la tyrosine, de l'aluminium, des saponines telles que Stimulon™, et des cytokines peuvent également être ajoutés pour améliorer l'efficacité de la composition.
Les séquences ARN de l'invention peuvent être préparées avec un agent, tels que des microsphères injectables, des particules bio-érodables, des composés polymériques (tels que l'acide polylactique ou l'acide polyglycolique), des billes ou des liposomes qui peuvent fournir une libération contrôlée ou prolongée du produit.

Des exemples de supports acceptables sur le plan diététique comprennent par exemple des sucres, des saponines, etc.
La composition peut aussi inclure un ou plusieurs autres principes actifs choisis de préférence parmi un agent utilisable dans le traitement d'une laminopathie, en particulier dans le traitement du syndrome de Hutchinson-Gilford ou HGPS (connu aussi sous le nom de progéria et qui consiste en un vieillissement précoce et accéléré de l'être humain), de l'obésité, du diabète, du cancer, d'une maladie dégénérative, en particulier neurodégénérative (par exemple une leucodystrophie ou la paraplégie spastique héréditaire encore identifiée en tant que SPG35), d'un stress ou de l'infertilité. Un principe actif dans le traitement de l'obésité peut être par exemple la leptine ou l'un de ses dérivés.
Un principe actif dans le traitement du diabète peut être par exemple l'insuline ou l'un de ses dérivés
Un principe actif dans le traitement du cancer peut être par exemple un agent de chimiothérapie cytotoxique conventionnelle choisi parmi un inhibiteur de la réplication de l'ADN (tels que des agents liant l'ADN en particulier des composés intercalants ou alkylants), un agent anti-métabolite (tels que des inhibiteurs de l'ADN polymérase ou un inhibiteur de la topoisomérase I ou II), un agent anti-mitogénique (par exemple un alcaloïde), et un agent bloquant la croissance d'une tumeur cancéreuse (tels qu'un inhibiteur de la tyrosinase ou un anticorps monoclonal).

L'ARN selon l'invention (éventuellement compris ou exprimé à l'aide d'un autre produit selon l'invention décrit dans le présent texte) et le ou les autres composés actifs, peuvent être administrés simultanément, le cas échéant au sein d'une même composition, ou séquentiellement.

Les produits de l'invention (séquences d'acide nucléiques, vecteurs, cellules, compositions) peuvent être adaptés pour une administration par voie intraveineuse, orale, sublinguale, parentérale, rectale, topique, transdermique, sous-cutanée, muqueuse, intramusculaire, intrapulmonaire, intranasale, vaginale, etc., selon des protocoles classiques bien connus de l'homme du métier.
De manière préférée, le produit est adapté pour une administration par voie orale et se présente sous une forme solide ou liquide. Le produit peut se présenter par exemple sous la forme d'un aliment, d'un comprimé, d'une gélule, d'une pilule, d'une dragée, d'une pastille, de granules, ou d'une solution buvable. Dans le cas d'une administration par voie parentérale, il se présente de préférence sous la forme d'une solution liquide, d'une émulsion ou d'une suspension. Dans le cas d'une administration par voie intraveineuse, intradermique ou sous cutanée, il se présente typiquement sous la forme d'une solution injectable.
La séquence nucléotidique d'intérêt peut également être administrée par électroporation, dans les muscles ou à travers la peau du sujet.

Le dosage (ou la quantité efficace sur le plan thérapeutique) est facilement déterminé par l'homme du métier pour que les effets désirés (i.e. prolongation de la durée de vie, lutte contre les effets délétères du vieillissement, contre un stress et/ou contre l'infertilité) soient atteints. La dose spécifique de produit devra être adaptée au sujet concerné et à l'application souhaitée. Elle dépend d'une pluralité de facteurs dont le poids, l'état de santé, le sexe, le régime alimentaire du sujet, la voie d'administration, les taux d'absorption et d'excrétion, et l'éventuelle combinaison avec une ou plusieurs autres molécules actives.
La dose journalière totale du produit administrée à un sujet humain en une seule dose ou en plusieurs doses peut être, par exemple, comprise entre environ 1µg et 20mg par kilo, de préférence entre 100µ et 5mg. Les administrations peuvent être hebdomadaires ou quotidiennes voire répétées plusieurs fois par jour. Les ARNs ou compositions selon l'invention en comprenant peuvent être administrés sous forme de dose unitaire comprenant de 0,05 à 20 mg d'ARN, préférentiellement de 0,1 à 5 mg.

Dans un mode de réalisation particulier, une séquence ARN d'intérêt de l'invention est utilisée dans une composition thérapeutique, par exemple vaccinale.

Dans un autre mode de réalisation particulier, une séquence ARN d'intérêt de l'invention est utilisée dans une composition cosmétique.

### Applications des produits de l'invention

La présente invention concerne en particulier un produit tel que décrit dans le présent texte consistant en, ou comprenant, une séquence ARN ou une séquence ADN d'intérêt selon l'invention pour une utilisation comme médicament.
Elle concerne également un produit ou une composition selon l'invention pour une utilisation i) pour prévenir ou traiter une pathologie, une anomalie, un désordre ou une détérioration apparente ou fonctionnelle lié(e) à un mécanisme du vieillissement, ii) pour prolonger la durée de vie d'un sujet, ou iii) pour augmenter la résistance d'un sujet à un stress.
Dans un aspect particulier, la présente invention concerne l'utilisation d'un tel produit en une quantité efficace sur le plan thérapeutique pour la préparation d'une composition pharmaceutique destinée i) à la prévention ou au traitement d'une pathologie, d'une anomalie, d'un désordre ou d'une détérioration apparente ou fonctionnelle lié(e) à un mécanisme du vieillissement, ii) au prolongement de la durée de vie d'un sujet, ou iii) à l'augmentation de la résistance d'un sujet à un stress.
Dans encore un autre aspect particulier, la présente invention concerne l'utilisation d'un tel produit pour la préparation d'une composition cosmétique.

Le terme « traitement » tel qu'utilisé dans ce document, se réfère à une amélioration ou disparition des symptômes, un ralentissement de la progression de la maladie ou du processus de vieillissement, un arrêt de l'évolution de la maladie ou une disparition de la maladie.

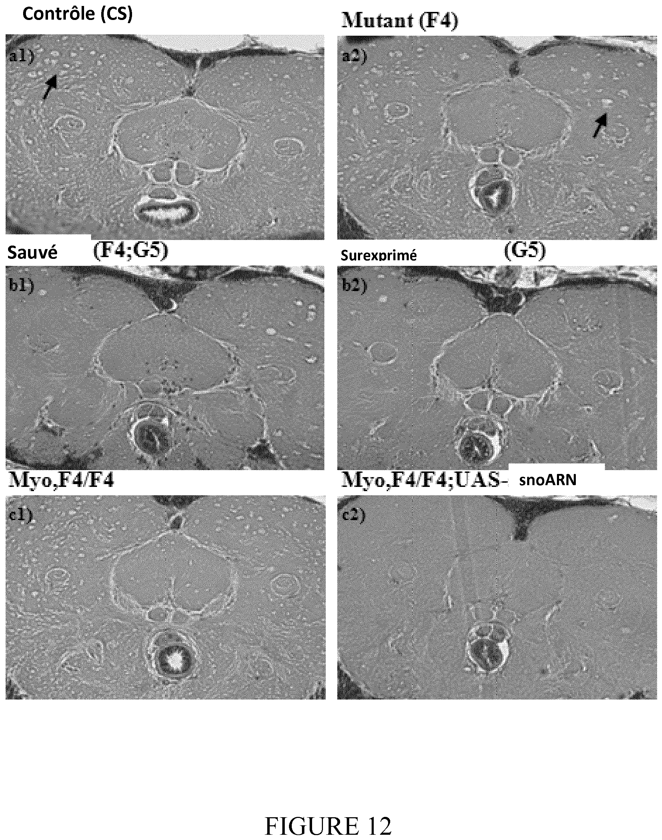

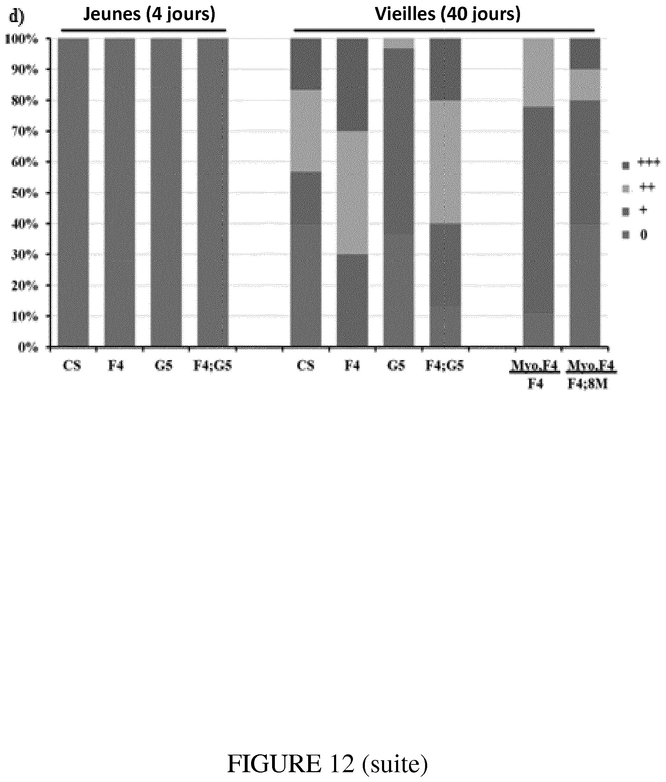

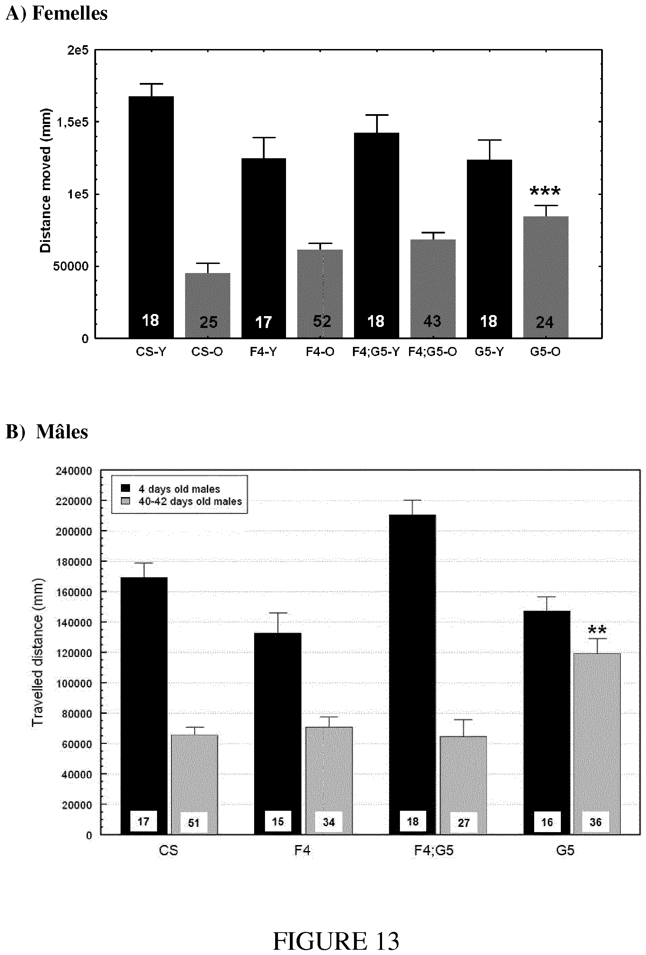

Les inventeurs ont mis en évidence l'efficacité surprenante des séquences ARN selon l'invention dans le retardement du ou des processus de vieillissement et la lutte contre les pathologies et effets délétères associés à ce(s) processus.
Les inventeurs ont notamment apporté la démonstration que la surexpression chez la drosophile du snoARN:Ψ28S-1153 de séquence SEQ ID NO :1 double de façon spectaculaire la durée de vie de ladite drosophile. Ils ont en particulier démontré que les mouches mutantes pour le snoARN:Ψ28S-1153 présentent plus de lésions neuro-dégénératives que les mouches de type sauvage à 40 jours d'âge (Figure 12). En outre, les mouches sur-exprimant le snoARN:Ψ28S-1153 ont moins de, voire aucune, lésions au cerveau, ce qui révèle le caractère protecteur du snoARN selon l'invention vis-à-vis des pathologies neurodégénératives. Par ailleurs, les inventeurs ont montré que les mouches âgées de 40 jours sur-exprimant le snoARN:Ψ28S-1153 présentent de meilleures performances sensori-motrices (représentées ici par la distance parcourue lors d'un test quantifiant l'activité locomotrice) que les mouches sauvages et sont ainsi protégées des effets délétères associés aux mécanismes du vieillissement (Figure 13).

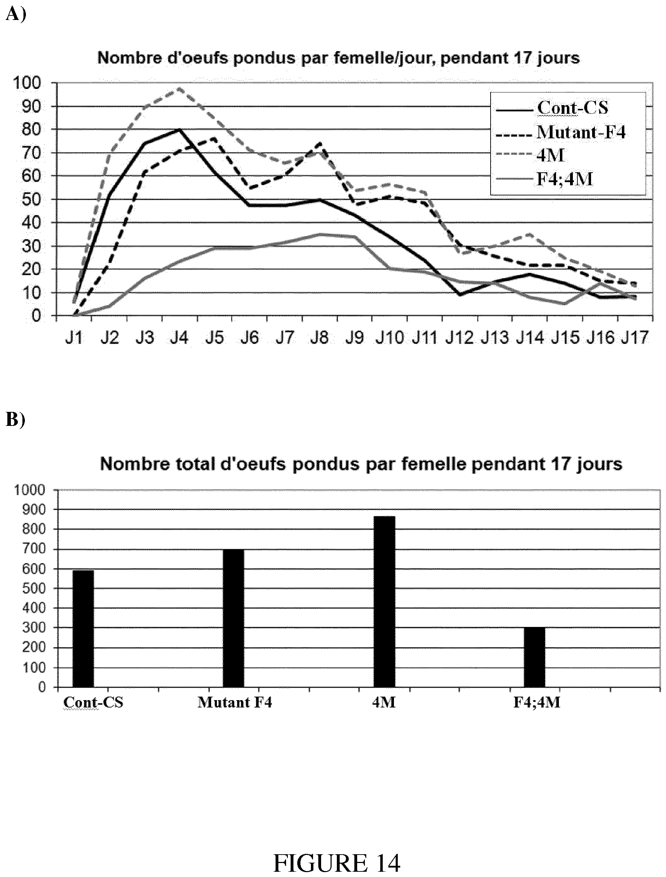

Les inventeurs ont apporté la démonstration que la sur-expression d'une séquence ARN d'intérêt selon l'invention permet de prolonger la durée de vie d'un sujet, de retarder les mécanismes du vieillissement et de lutter contre les effets délétères associés à ces mécanismes. Ils ont notamment démontré que cette surexpression est neuroprotectrice et permet en particulier de prévenir ou de traiter une maladie dégénérative, en particulier neurodégénérative, typiquement les maladies neurodégénératives associées à l'accumulation de fer qui entraînent une démyélinisation dans le cerveau telles que par exemple les maladies neurodégénératives provoquées par une ou plusieurs mutations du gène fa2h. L'invention permet en particulier de traiter des leucodystrophies (typiquement celles liées aux mutations du gène fa2h) ou une paraplégie spastique héréditaire (typiquement la paraplégie spastique héréditaire SPG35). Elle permet par ailleurs de prévenir ou de traiter le diabète, l'obésité, un cancer, ou de lutter contre un problème de fertilité ou stérilité (Figure 14), et favorise en outre le maintien de l'activité locomotrice du sujet traité.

Par « *sujet* » on entend tout être vivant susceptible de bénéficier d'un tel traitement, quelque soit son sexe ou son âge. Préférablement, le sujet est un sujet adulte. Le sujet peut être un insecte tel qu'une drosophile, ou un animal, par exemple un mammifère (de préférence un primate ou un être humain) ou un rongeur (de préférence une souris ou un rat).
De manière préférée, le sujet i) n'exprime pas la séquence ARN d'intérêt ou exprime une version anormale de ladite séquence ARN, ii) est un sujet soumis à des conditions de stress (en particulier un sujet ayant atteint sa maturité sexuelle, de préférence un sujet âgé) telles que le jeûne, un choc thermique, un stress oxydatif (comme une exposition au Paraquat), ou un stress (au niveau de l'intestin) responsable d'une prolifération cellulaire détectable ou d'une dérégulation de la voie Delta/Notch, et/ou iii) est un sujet souffrant d'une maladie liée à ou favorisée par la vieillesse, typiquement d'une maladie dégénérative, en particulier neurodégénérative, d'une laminopathie telle que la progéria, d'obésité, de diabète, d'un cancer ou d'un problème de fertilité (comprenant notamment l'infertilité ou la stérilité).
Dans un mode de réalisation particulier, le génome du sujet susceptible de bénéficier de l'invention i) n'exprime pas une séquence ARN d'intérêt telle que décrite dans le présent texte, ii) exprime une version anormale de ladite séquence ARN d'intérêt, ou iii) contient une séquence ADN comprenant une mutation responsable de la non expression ou d'une expression anormale (i.e. non fonctionnelle) de ladite séquence ARN d'intérêt.
La version normale (sauvage) de la séquence ADN peut être choisie par exemple parmi les séquences SEQ ID NO : 5, SEQ ID NO : 6, SEQ ID NO : 7, SEQ ID NO : 8, SEQ ID NO : 10, SEQ ID NO : 12, SEQ ID NO : 14, SEQ ID NO : 16, SEQ ID NO : 18, SEQ ID NO : 20, SEQ ID NO : 22, SEQ ID NO : 24, SEQ ID NO : 26, SEQ ID NO : 28 et SEQ ID NO : 30, identifiées précédemment. Une mutation susceptible d'affecter la version normale de la séquence ADN, i.e. d'empêcher l'expression d'une séquence ARN d'intérêt selon l'invention, est typiquement choisie parmi une délétion, une addition et/ou une substitution d'un ou de plusieurs nucléotides.

Le terme « *longévité* » ou « *durée de vie* » se réfère à la durée de vie pour laquelle un sujet est programmé en tant qu'espèce biologique, à condition de vivre dans des conditions idéales et en l'absence de maladie ou d'accident. La « *longévité maximale* » ou « *durée de vie maximale »* correspond à la durée de vie maximale que pourraient atteindre les individus d'une espèce donnée. Par soucis pratique, on estime la longévité maximale d'un sujet par l'âge maximal atteint par un des membres d'une population. La « *longévité moyenne* » ou « *durée de vie moyenne* » est l'âge auquel 50% d'une population donnée survit.
Une méthode largement reconnue pour mesurer l'effet d'un traitement sur la *« prolongation de la durée de vie* » est par référence à la longévité maximale ou à la longévité moyenne. Si un traitement augmente significativement la longévité maximale ou moyenne d'un groupe de sujet par rapport à un groupe de sujets contrôles, alors on considère qu'il y a une prolongation de la durée de vie et un retardement du vieillissement.
Chez la drosophile, la maturité sexuelle est atteinte, en moyenne, à l'âge de un jour. La durée de vie moyenne est d'environ 30 jours. La durée de vie maximale est comprise entre environ 60 et 80 jours selon l'espèce ou la lignée de Drosophile et les conditions de vie ou d'élevage. Chez la souris, la maturité sexuelle est atteinte, en moyenne, 42 jours après la naissance. La durée de vie moyenne est de 832 jours. Chez l'être humain, la maturité sexuelle est atteinte entre 9 et 14 ans. La durée de moyenne est de 73 ans pour les hommes et de 79 ans pour les femmes. La durée de vie maximale est à ce jour de 122 ans, 5 mois et 14 jours.
Le terme « *vieillissement* », dans le contexte de la présente invention, est un processus de changements graduels et spontanés allant de la maturation, via l'enfance et la puberté, jusqu'au déclin des fonctions du sujet. Le vieillissement comprend donc la composante positive du développement et de la maturation et la composante négative du déclin.

Les séquences ARN d'intérêt de l'invention augmentent la longévité (ou la durée de vie) maximale ou moyenne d'un sujet. De manière préférée, ces séquences prolongent la durée de vie et retardent le vieillissement d'un sujet ou d'une sous-population de sujets adultes, définie par exemple par le sexe et/ou l'âge (c'est-à-dire par un âge minimum ou par un âge maximum ou par une fenêtre d'âges).
A l'aide de la présente invention, il est typiquement envisageable de prolonger la durée de vie de la Drosophile, mais également de mammifères incluant la souris et en particulier l'être humain, d'environ 50% voire 100%, tout en améliorant les conditions de vieillissement.

A l'aide des séquences ARN de la présente invention il est également possible de lutter contre les « effets délétères du vieillissement ». Dans le contexte de la présente invention, cette expression désigne la composante négative du vieillissement, c'est à dire le déclin du sujet. Le déclin progressif, avec l'âge, des capacités physiologiques varie d'un organe à un autre et d'un sujet à un autre. Le déclin physiologique d'un sujet entraine une capacité réduite à répondre à un stimulus de l'environnement et une augmentation de la susceptibilité et de la vulnérabilité aux maladies et altérations. Les effets délétères du vieillissement englobent l'accumulation des changements indésirables ayant un impact sur l'affaiblissement d'un sujet donné et susceptibles de précipiter sa mort. Ces changements peuvent être attribués au développement, au processus de vieillissement inné, à d'éventuelles anomalies génétiques, à l'environnement, ainsi qu'aux maladies affectant ou ayant affecté le sujet.

Il est par ailleurs possible de prévenir ou traiter, à l'aide de la présente invention, des maladies dégénératives, en particulier neurodégénératives, typiquement de maladies dégénératives affectant le système nerveux central et/ou périphérique.
Dans un mode de réalisation préféré, l'invention permet de prévenir ou traiter les maladies neurodégénératives associées à l'accumulation de fer qui entraînent une démyélinisation dans le cerveau, en particulier les maladies neurodégénératives provoquées par une ou plusieurs mutations du gène fa2h. L'invention permet ainsi de traiter des leucodystrophies (typiquement celles liées aux mutations du gène fa2h) ou une paraplégie spastique héréditaire (typiquement la paraplégie spastique héréditaire SPG35).
Dans d'autres modes de réalisation, l'invention permet de prévenir ou traiter une pathologie sélectionnée parmi une myopathie, la maladie de Hunter, la dyskératose congénitale, le syndrome de Prader-Willi, la maladie d'Alzheimer, la maladie de Parkinson, la maladie de Huntington, la sclérose en plaques, la sclérose latérale amyotrophique (SLA).

Dans un autre mode de réalisation, la présente invention permet de prévenir ou traiter des laminopathies telles que le syndrome de Hutchinson-Gilford (ou progéria), la dysplasie Mandibuloacral (MAD), la dystrophie musculaire de Emery-Dreifuss, le syndrome atypique de Werner, la dermatophatie restrictive, l'akinésie fetale léthale, et la LIRLLC (Generalized lipoatrophy, insulin-resistant diabetes, leukomelanodermic papules, liver steatosis, and hypertrophic cardiomyopathy), de préférence le syndrome de Hutchinson-Gilford.

Dans encore un autre mode de réalisation, la présente invention permet de prévenir ou traiter le diabète ou l'obésité.

Il est également aussi possible de participer la prévention ou au traitement de cancer ou processus néoplasique. Le cancer est typiquement un cancer sélectionné parmi un carcinome, un sarcome, un lymphome, un mélanome, une tumeur pédiatrique, une leucémie. Il peut s'agir par exemple d'un cancer du sein, de l'ovaire, de l'endomètre, de la prostate, de l'oesophage, du colon, du rectum, du rein, du poumon, de la thyroïde, d'un ostéosarcome, d'un mélanome, d'une leucémie, d'un neuroblastome, etc.
Dans un mode de réalisation particulier, la présente invention est particulièrement utile pour lutter contre un cancer induit par les radicaux libres tels que le cancer du colon et le cancer de l'estomac.

Il est par ailleurs possible de lutter contre les problèmes d'infertilité ou de stérilité à l'aide d'un produit selon l'invention. Par « *infertilité* » ou « *stérilité* », on entend la difficulté, voire l'inaptitude, à donner la vie. Dans le cadre de cette utilisation, le sujet est typiquement une femelle, arrivée à maturation sexuelle et ayant des difficultés, par exemple prématurées, à se reproduire. Typiquement, le sujet est stérile, peu fertile, ou présente une diminution précoce ou prématurée de sa fertilité. Les séquences ARN d'intérêt selon l'invention permettent de traiter l'infertilité ou de restaurer, augmenter, renforcer ou stimuler la fertilité du sujet traité.

La présente invention concerne également une méthode de traitement d'une pathologie, d'une anomalie, d'un désordre, d'un effet délétère ou d'une détérioration apparente ou fonctionnelle lié(e) à un mécanisme du vieillissement ou à un stress, comme décrit dans le présent texte.

### Méthodes permettant d'évaluer l'efficacité préventive ou thérapeutique d'un composé test

Les inventeurs décrivent également une méthode permettant d'évaluer l'efficacité cosmétique ou thérapeutique d'un composé test pour lutter contre les effets délétères du vieillissement ou d'un stress ainsi que contre les pathologies associées à un tel stress ou à un mécanisme du vieillissement, ou pour lutter contre l'infertilité.

Par « *composé test* » on entend tout composé potentiellement impliqué dans la lutte contre les effets délétères du vieillissement, contre un stress ou contre l'infertilité. Il peut s'agir par exemple d'une molécule naturelle ou chimique, d'un métal, d'un agent irradiant, etc.

Un composé peut être testé pour son éventuelle efficacité thérapeutique, i.e., sa capacité à prévenir, traiter ou favoriser le traitement des pathologies et effets délétères du vieillissement ou d'un stress, par exemple une laminopathie, une maladie dégénérative ou un cancer tels que décrits précédemment, pour prévenir, traiter ou favoriser le traitement du diabète ou l'obésité, ou encore pour prévenir, traiter ou favoriser le traitement de l'infertilité.
Un composé peut autrement être testé pour son éventuelle efficacité cosmétique, i.e., sa capacité à améliorer l'apparence physique du sujet et à ainsi lutter contre une détérioration apparente du sujet liée à un mécanisme du vieillissement. Le composé test peut par exemple se révéler efficace dans le traitement cosmétique de l'épiderme, du système pileux et/ou capillaire, des ongles, lèvres, organes génitaux externes, dents et muqueuses.

Une méthode décrite comprend les étapes suivantes :
i) l'exposition d'une cellule, d'une population de cellules ou d'un tissu, n'exprimant pas la séquence d'ARN selon l'invention ou exprimant une version anormale de ladite séquence d'ARN ; ou d'une souris transgénique selon l'invention,
ii) l'évaluation des effets, s'ils existent, du composé test sur le phénotype de ladite ou desdites cellules ou dudit tissu ou de ladite souris transgénique, et
iii) la détermination de l'efficacité cosmétique ou thérapeutique dudit composé test, une restauration de l'activité et/ou de l'expression de ladite séquence d'ARN étant corrélée à une efficacité dudit composé test.

L'efficacité cosmétique ou thérapeutique dudit composé test peut être évaluée ou déterminée en référence à une valeur contrôle. Cette valeur contrôle peut être établie à partir du niveau d'activité et/ou d'expression de ladite séquence ARN dans une cellule, une population de cellules, ou un tissu n'exprimant pas, ou exprimant une version anormale de ladite séquence d'ARN ou dans une souris transgénique selon l'invention.
Si l'activité et/ou l'expression de ladite séquence d'ARN présente dans la cellule, dans la population de cellules, dans le tissu ou dans la souris transgénique exposée(s) au composé test est supérieure à l'activité et/ou l'expression de la séquence d'ARN dans la cellule, la population de cellules ou le tissu n'exprimant pas, ou exprimant une version anormale de ladite séquence d'ARN ou dans la souris transgénique de l'invention, alors le composé test possède une efficacité cosmétique ou thérapeutique.

Une deuxième méthode décrite comprend les étapes suivantes :
i) l'exposition d'une cellule selon l'invention comprenant une séquence d'ARN selon l'invention, d'une population de cellules ou d'un tissu comprenant une cellule selon la l'invention,
ii) l'évaluation des effets, s'ils existent, du composé test sur le phénotype de ladite cellule, de ladite population de cellules ou dudit tissu, et
iii) la détermination de l'efficacité cosmétique ou thérapeutique dudit composé test, une augmentation de l'activité et/ou de l'expression de ladite séquence d'ARN étant corrélée à une efficacité dudit composé test.

Selon cette méthode, une valeur contrôle peut être établie à partir du niveau d'activité et/ou d'expression de ladite séquence d'ARN dans une cellule ou une population de cellules exprimant ladite séquence d'ARN
Dans ce cas, si l'activité et/ou l'expression de ladite séquence d'ARN présente dans la cellule, ou dans la population de cellules exposée(s) au composé test est supérieure à l'activité et/ou l'expression de la séquence d'ARN dans la cellule ou la population de cellules exprimant ladite séquence d'ARN, alors le composé test possède une efficacité cosmétique ou thérapeutique.
La quantification du niveau d'activité et/ou d'expression de la séquence d'ARN peut se faire à l'aide de techniques connues de l'homme du métier, typiquement par PCR quantitative ou immuno-histochimie (coloration des anticorps dirigés contre les gènes/protéines régulés par le snoARN d'intérêt).
L'évaluation de l'effet thérapeutique peut autrement se faire par exemple par détermination de la durée de vie des cellules, par la mesure du nombre de divisions cellulaires, etc.

De manière préférée, la cellule ou la population de cellules exposée(s) au composé test est d'origine intestinale ou ovarienne. Des cellules préférées peuvent être choisies parmi les cellules mentionnées dans le présent texte, typiquement parmi les entéroblastes, entérocytes, cellules entéro-endocrines, cellules souches intestinales, cellules nourricières, typiquement cellules nourricières de la chambre ovarienne, ovocyte, ovoblaste, etc.

D'autres aspect et avantages de la présente invention apparaîtront à la lecture des figures et exemples qui suivent, qui doivent être considérés comme illustratifs et non limitatifs.

### LEGENDES DES FIGURES

**Figure 1** **:** carte de la région génomique du locus P[Gal4]4C, positionnement du snoARN *jouvence* et de la délétion F4.
**Figure 2** **:** A) motif de la boîte H/ACA (« H/ACA box »), B) Schéma de la pseudo-uridinylation. (Extrait de : Kiss et al., (2010) Molecular Cell, 37, 597-606).
**Figure 3** **:** Longévité des mouches femelles. Cumulative décroissante (en %) du nombre de mouches vivantes en fonction du nombre de jours. Cont-CS= mouches contrôle de type sauvage. F4= mutant F4, G5= mouches portant le transgène génomique du snoARN G5, F4;G5= mouches portant le transgène génomique du snoARN G5 dans le fond génétique mutant F4, pour restaurer le phénotype sauvage (entre parenthèses: le nombre de mouches).
**Figure 4** **:** test de résistance aux stress (choc thermique, jeûne, paraquat) pratiqué sur des mouches âgées de 3 jours.
   A) Choc Thermique : Les mutants F4 sont plus résistants que les contrôles (femelles seulement). Les mouches sur-exprimant le snoARN (G5), ainsi que les mouches mutantes F4 pourvues du transgène (F4;G5) sont plus résistantes que les mouches contrôles (mâles et femelles).
   B) Jeûne : Les mutants F4 sont plus résistants que les contrôles (mâle et femelles). Les mouches sur-exprimant le snoARN (G5) sont plus résistantes (femelles seulement). Les mouches femelles mutantes F4 pourvues du transgène (F4;G5) sont similaires aux mouches contrôles, donc il y a restauration du phénotype sauvage par le transgène.
   C) Paraquat : Les mutants F4 sont plus résistants que les contrôles (mâle et femelles). Les mouches sur-exprimant le snoARN (G5) sont plus résistantes (femelles seulement). Les mouches mutantes F4 pourvues du transgène (F4;G5) sont similaires aux mouches contrôles, donc il y a restauration du phénotype sauvage par le transgène.

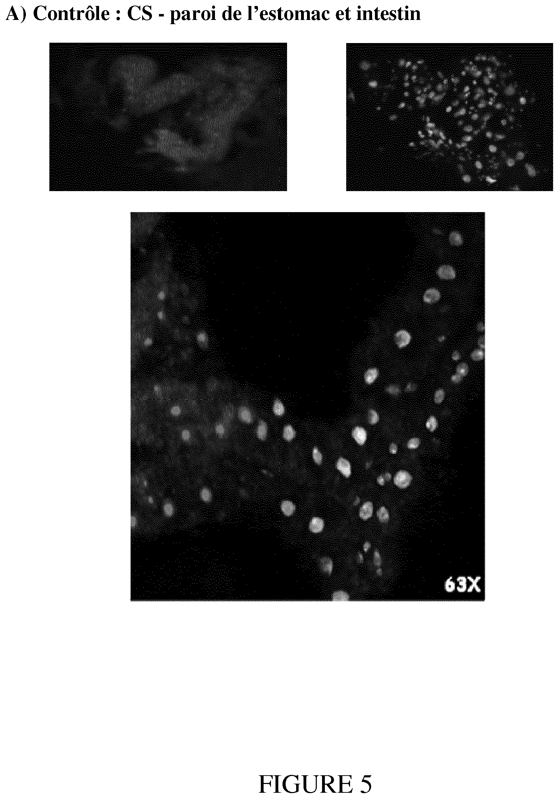

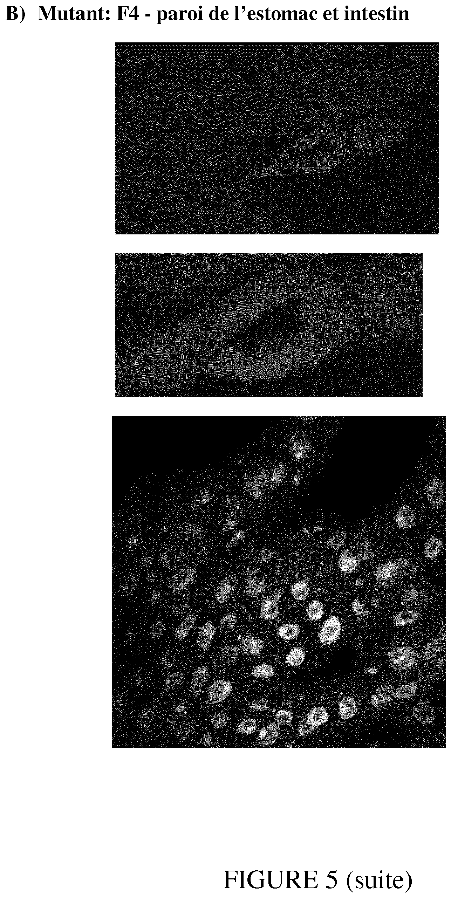

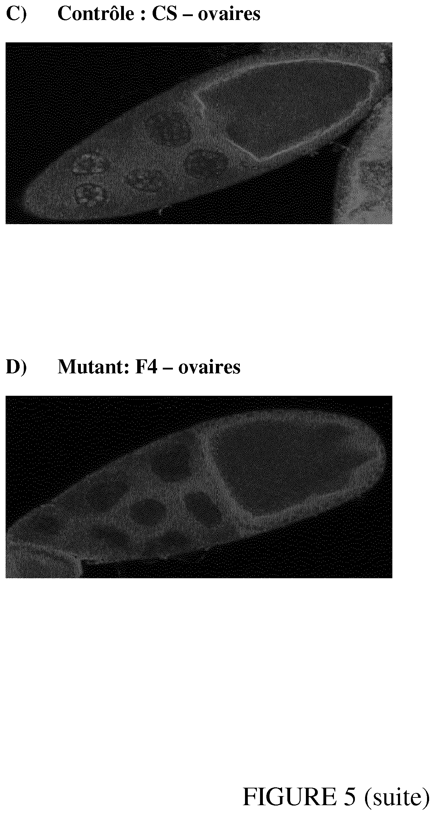

**Figure 5** **:** Hybridation *in situ* sur l'intestin et les ovaires.
   A) sur les mouches contrôles, type sauvage (Canton-S), le snoARN est exprimé dans les cellules épithéliales de l'intestin. En bleu : coloration de l'ADN dans le noyau par le dapi. En rouge : coloration du snoARN. On remarque que la coloration du snoARN (points rouge) est distincte et complémentaire de celle du noyau (bleu), démontrant que ce dernier est localisé dans le nucléole.
   B) dans le mutant F4 (délétion du snoARN), on voit la coloration en bleu (dapi) mais aucune coloration en rouge, car le snoARN est délété, et donc n'est pas exprimé.
   C) mouches contrôles, type sauvage (Canton-S). Le snoARN est exprimé dans les cellules nourricières de l'ovaire.
   D) mouches mutantes F4. Le snoARN n'est pas exprimé dans les ovaires.

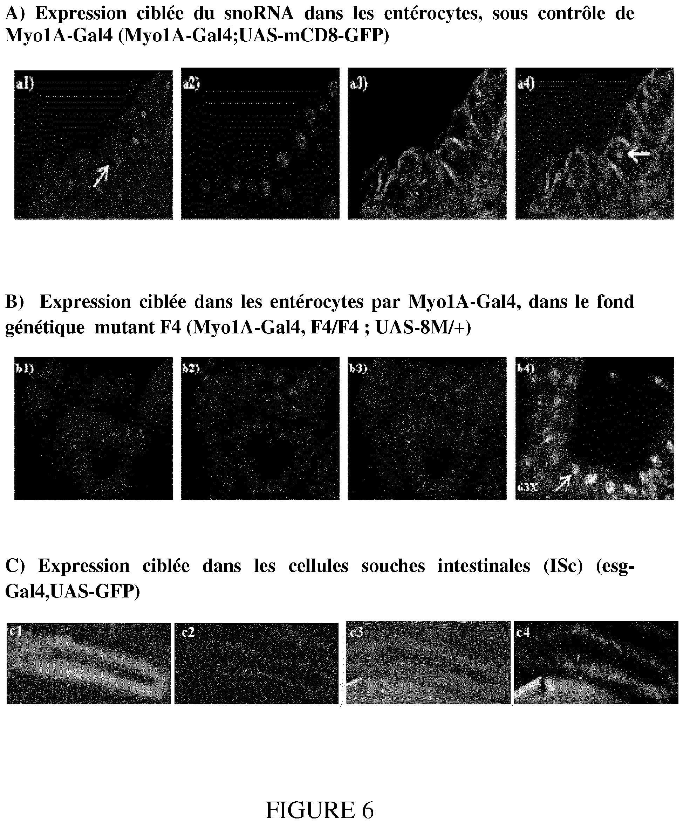

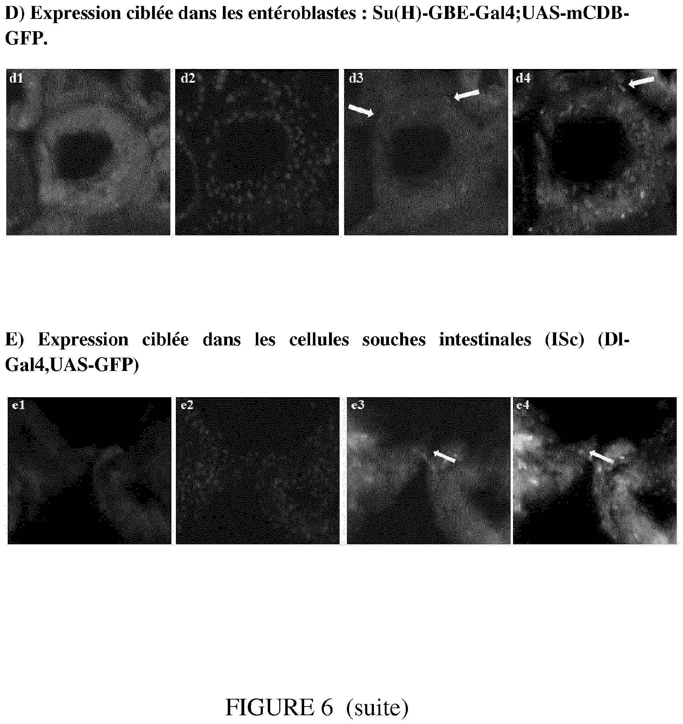

**Figure 6** **:** Expression ciblée du snoARN dans les cellules épithéliale de l'intestin, via quatre lignées pilotes Gal4 distinctes, pilotant l'expression d'un gène rapporteur GFP (UAS-GFP). Détection de l'expression du snoARN par hybridation *in-situ* (en rouge et/ou orange) (colonne de gauche : a1,b1,c1,d1,e1), de la coloration du noyau par le dapi (en bleu) (deuxième colonne : a2,b2,c2,d2,e2), de l'expression de la GFP par immuno-marquage (anticorps anti-GFP révélé par la FITC (en vert) (troisième colonne: a3,b3,c3,d3,e3). Colonne de droite (a4,b4,c4,d4,e4) superposition des 3 images précédentes montrant la co-localisation. On peut constater un double marquage seulement en (A) (Myo1A-Gal4), démontrant que le snoARN n'est exprimé que dans les entérocytes. A) Expression ciblée du snoARN dans les entérocytes, sous contrôle de Myo1A-Gal4 (Myo1A-Gal4;UAS-mCD8-GFP). B) Expression ciblée dans les entérocytes par Myo1A-Gal4, dans le fond génétique mutant F4 (Myo1A-Gal4, F4/F4 ;UAS-snoRNA-8M/+). C) Expression ciblée dans l'intestin sous contrôle de esg-Gal4 (esg-Gal4,UAS-GFP) marquant les cellules souches intestinales (ISCs). D) Expression ciblée dans l'intestin sous contrôle de Su(H)-Gal4 (Su(H)-GBE-Gal4;UAS-mCDB-GFP), marquant les entéroblastes. E) Expression ciblée dans l'intestin sous contrôle de Delta-Gal4 (Dl-Gal4,UAS-GFP), marquant les cellules souches intestinales (ISCs).

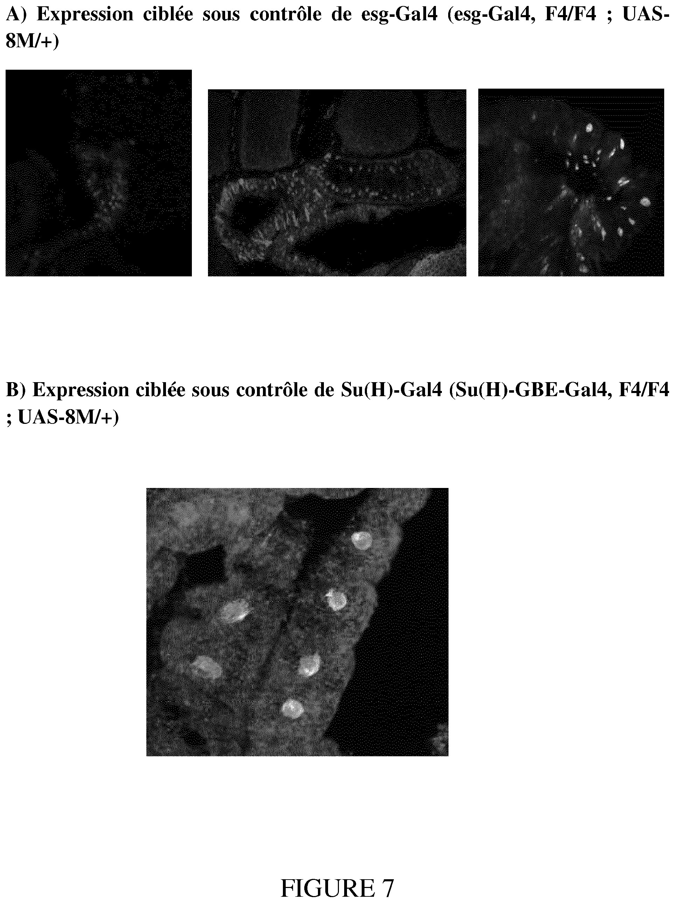

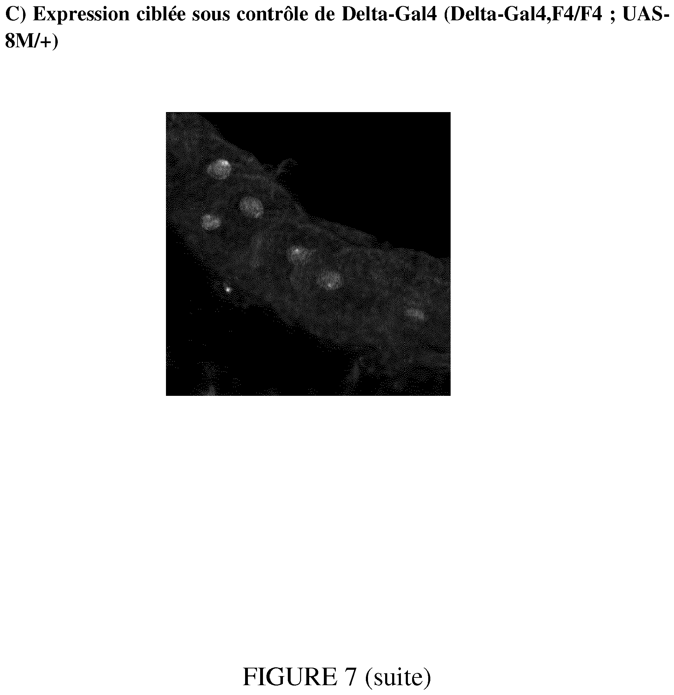

**Figure 7** **:** Expression ciblée du snoARN dans les autres types cellulaires de l'épithélium de l'intestin, via les trois autres lignées pilotes Gal4 distinctes, pilotant l'expression du snoARN (UAS-8M), démontrant que le snoARN peut être exprimé de façon ectopique dans d'autres types cellulaires.
   A) Expression ciblée sous contrôle de esg-Gal4 (esg-Gal4, F4/F4;UAS-8M/+)
   B) Expression ciblée sous contrôle de Su(H)-Gal4 (Su(H)-GBE-Gal4, F4/F4;UAS-8M/+).
   C) Expression ciblée sous contrôle de Delta-Gal4 (Delta-Gal4,F4/F4;UAS-8M/+).

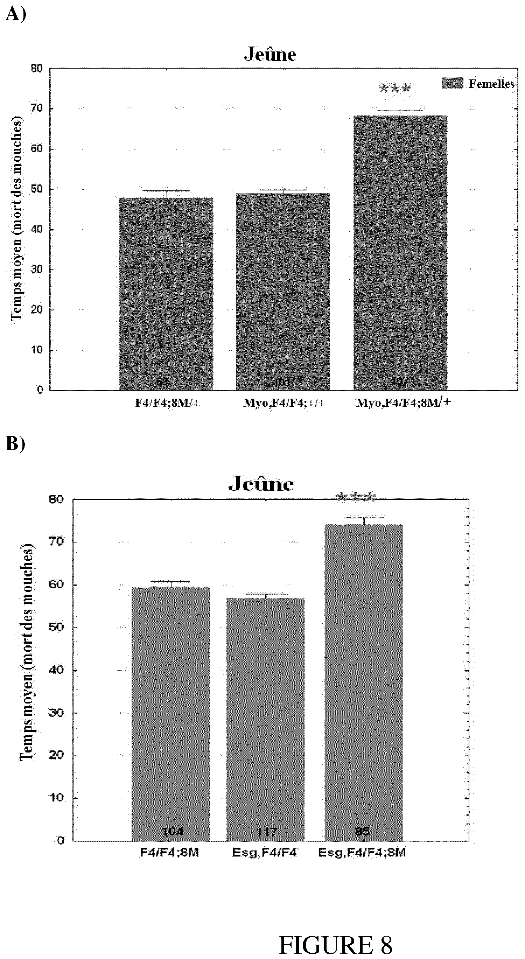

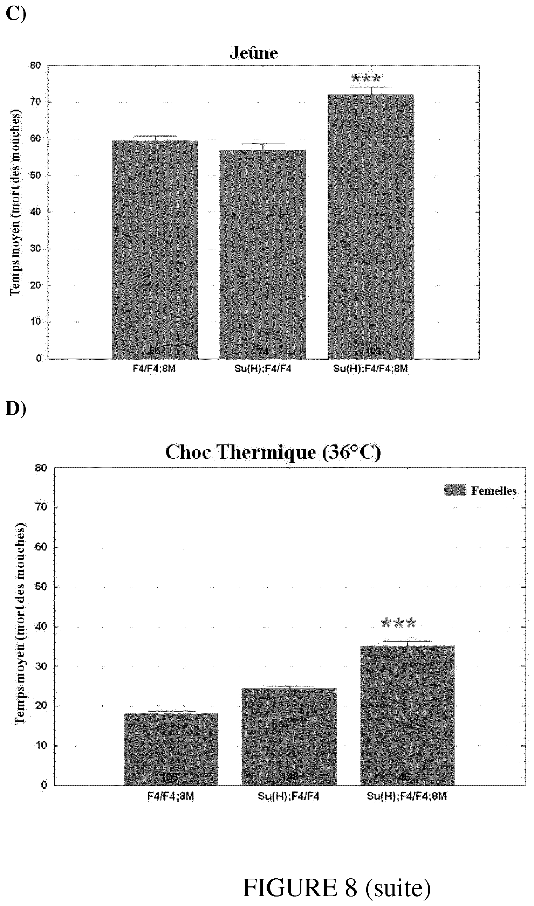

**Figure 8** **:** Résistance à divers stress suite à l'expression ciblée (re-expression du snoARN dans le fond génétique mutant F4) du snoARN dans l'intestin de mouches âgées de 3 jours.
   A) Jeûne : Expression ciblée du snoARN (UAS-8M) sous contrôle de Myo1A-Gal4. Il y a une nette augmentation de la résistance chez les mouches exprimant le snoARN (Myo,F4/F4 ; 8M/+) par rapport aux deux lignées contrôles.
   B) Jeûne : Expression ciblée du snoARN (UAS-8M) sous contrôle de esg-Gal4. Il y a une nette augmentation de la résistance chez les mouches exprimant le snoARN (esg,F4/F4 ; 8M) par rapport aux deux lignées contrôles.
   C) Jeûne : Expression ciblée du snoARN (UAS-8M) sous contrôle de Su(H)-Gal4. Il y a une nette augmentation de la résistance chez les mouches exprimant le snoARN (Su(H),F4/F4 ; 8M) par rapport aux deux lignées contrôles.
   D) Choc thermique à 36°C: Expression ciblée du snoARN (UAS-8M) sous contrôle de Su(H)-Gal4. Il y a une nette augmentation de la résistance chez les mouches exprimant le snoARN (Su(H),F4/F4;8M) par rapport aux deux lignées contrôles.
**Figure 9** **:** A) Homologies chez les douze espèces de Drosophile dont le génome est connu ; B) structure consensus des 12 espèces de Drosophile.

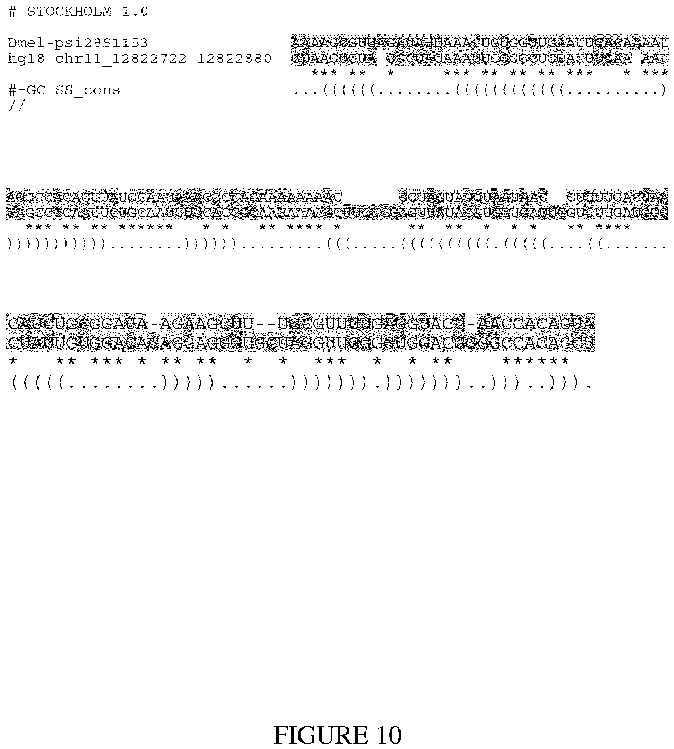

**Figure 10** **:** Homologue humain sur le chromosome 11.

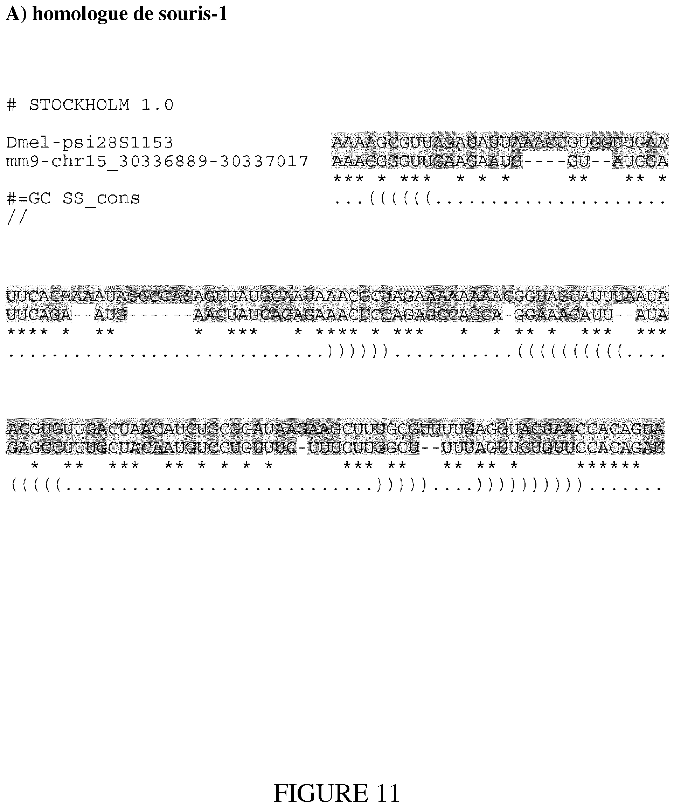

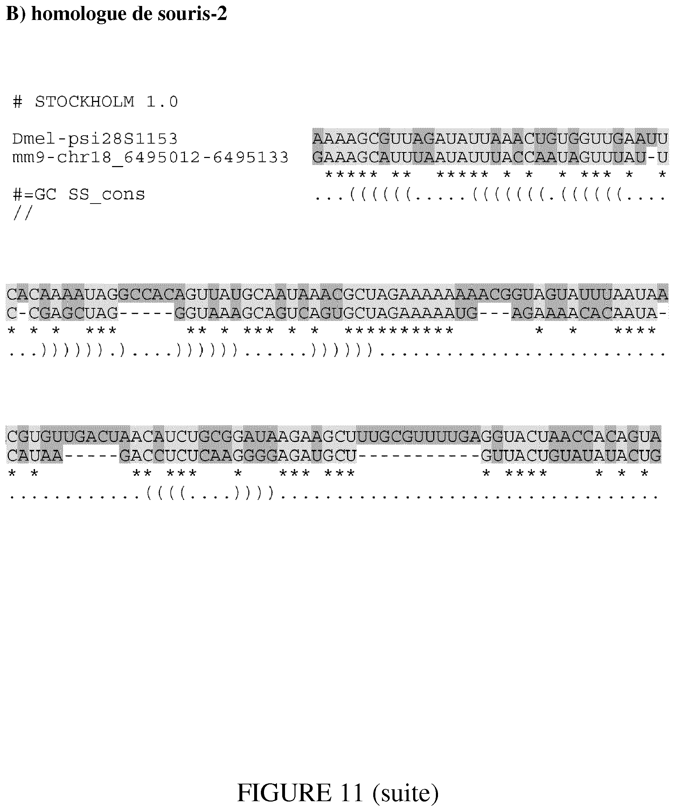

**Figure 11** **:** 2 gènes homologues chez *Mus musculus* (souris-1 et souris-2).
**Figure 12** **:** Histologie du cerveau : neuro-dégénérescence visible sur le cerveau de mouches âgées de 40 jours. Les mouches contrôles (type sauvage Canton-S) (a1) présentent des lésions de neurodégénérescence (vacuoles/trous), alors que les mouches mutantes F4 (a2) présentent plus de lésions. Les mouches exprimant le snoARN dans le fond génétique mutant (rescued : F4;G5) (b1) présentent moins de lésions que les CS et F4, ainsi que les mouches sur-exprimant le snoARN (G5) (b2). Les mouches mutantes F4 re-exprimant le snoARN spécifiquement dans les entérocytes (Myo,F4/F4 ;UAS-snoRNA) (c2) présentent également moins de lésions que celles ne l'exprimant pas (Myo,F4/F4) (c1). En (d), quantification des lésions chez les jeunes mouches (âgées de 4 jours) et les vieilles mouches (âgées de 40 jours), pour ces différents génotypes. Tous ces résultats démontrent que l'expression du snoARN protège contre les lésions neurodégénératives chez les vieilles mouches (0, +, ++, +++ = degré de sévérité des lésions basé sur leurs nombres et leur surface).
**Figure 13** **:** Un paramètre sensori-moteur (activité locomotrice) quantifié par video-tracking, représenté ici par la distance parcourue pendant 7 heures d'enregistrement. Les mouches âgées de 40 jours sont comparées à des mouches jeunes âgées de 4 jours. A) Distance parcourue par les femelles. B) Distance parcourue par les mâles.
**Figure 14** **:** Fertilité des mouches (nombre d'oeufs pondus A) par femelle/jour et B) par femelle en 17 jours). Au total, les mutants F4 pondent un peu plus d'oeufs que les contrôles (CS), mais avec un léger retard. L'expression du transgène (snoARN) dans le mutant F4 (F4;G4 - appelé aussi F4;4M) diminue la fertilité. La sur-expression du transgène (G4 - appelé aussi 4M) augmente la fertilité (nombre d'oeufs pondus).

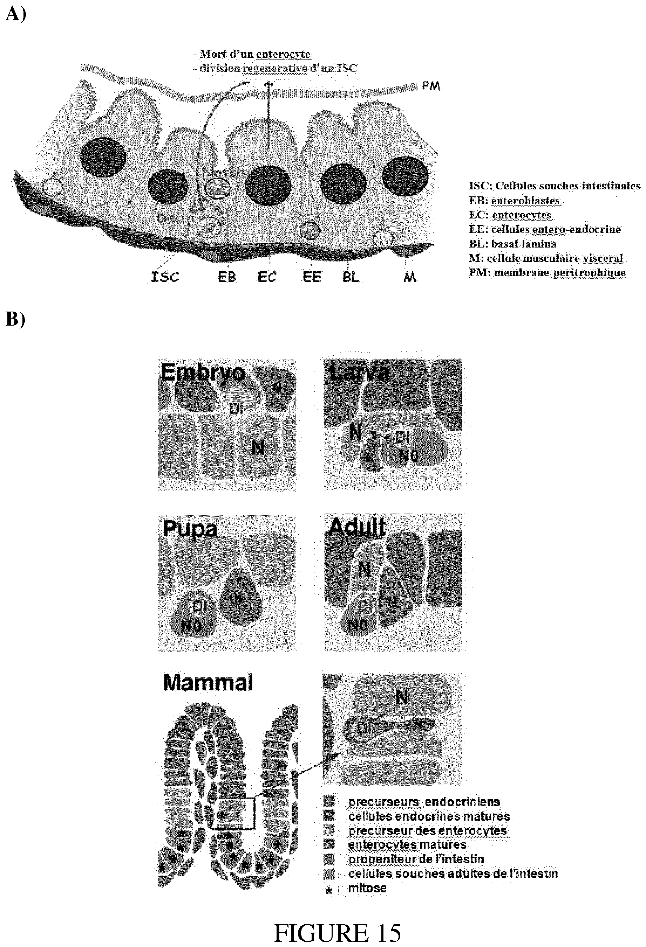

**Figure 15** **:** Schéma de l'épithélium intestinal (Extrait de : Jiang and Edgar, Exp. Cell. Res., 2011).
   A) Modèle de la régénérescence de l'épithélium intestinal chez la Drosophile adulte.
   B) Modèle de la fonction de Notch dans les cellules entero-endocrines chez la Drosophile (embryon, larve, pupe, adulte), et chez les mammifères. Dl= Delta, N= Notch, NO = absence de signal de Notch. Ce schéma montre clairement la similitude parfaite entre l'épithélium intestinal de la Drosophile et celui de l'homme.

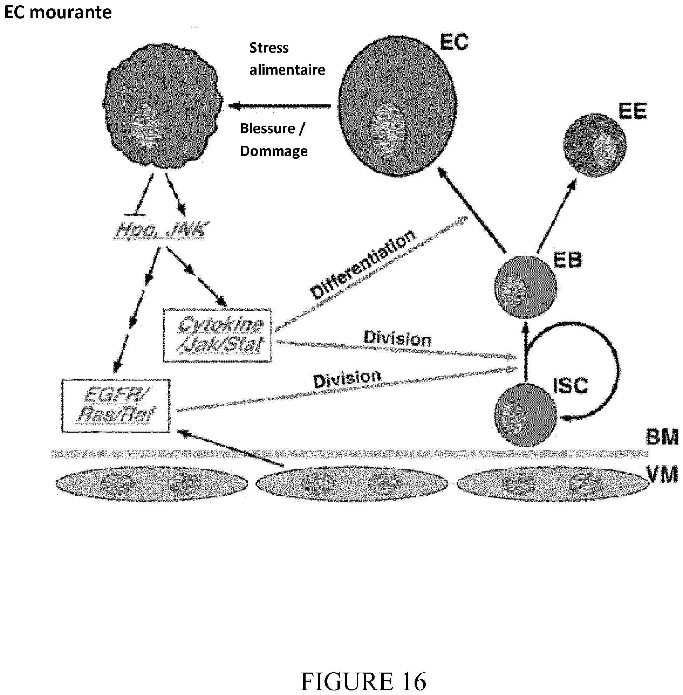

**Figure 16** **:** Mécanisme de rétro-contrôle régulant l'homéostasie et la régénération de l'intestin (midgut) chez la Drosophile (Extrait de: Jiang and Edgar, Exp. Cell. Res., 2011). (même abréviation que pour la Figure 15).
**Figure 17** **:** Le snoARN *jouvence* est impliqué dans l'épissage alternatif du gène CG9339 (*skywalker*). Des RT-PCR comparatifs ont été effectués sur certains gènes cibles potentiels afin de rechercher si dans le mutant F4, certains de ces gènes n'étaient pas épissés correctement, par rapport aux mouches Type Sauvage (Control-CS). Entre-autres, 4 transcrits de 345-400-432-462pb du gène CG9339 (*skywalker*) ont été analysés. Le gène rp49 (fragment de 300bp) est utilisé comme contrôle interne, ainsi qu'un contrôle avec ARN (sans RT au préalable) afin de démontrer que l'ARN utilisé n'est pas contaminé par des traces d'ADN. On remarque l'absence du petit transcrit de 345bp du gène CG9339 (*skywalker*) chez le mutant F4 (flèche).
**Figure 18** **:** Le snoARN *jouvence* est impliqué dans la régulation du niveau d'ARN de deux autres gènes : *klarsicht* et le gène CG30502 (*fa2h*). A) Pour le gène *klarsicht,* l'amplification d'un fragment de 464 pb (compris dans un exon) montre qu'il y a moins de transcrit de produit chez le mutant F4 que chez les mouches contrôles de type sauvage (Canton-S). B) Pour le gène *fa2h,* l'amplification d'un fragment de 429 pb (compris dans un exon) montre qu'il y a moins de transcrit chez le mutant F4 que chez les mouches contrôles de type sauvage (Canton-S).

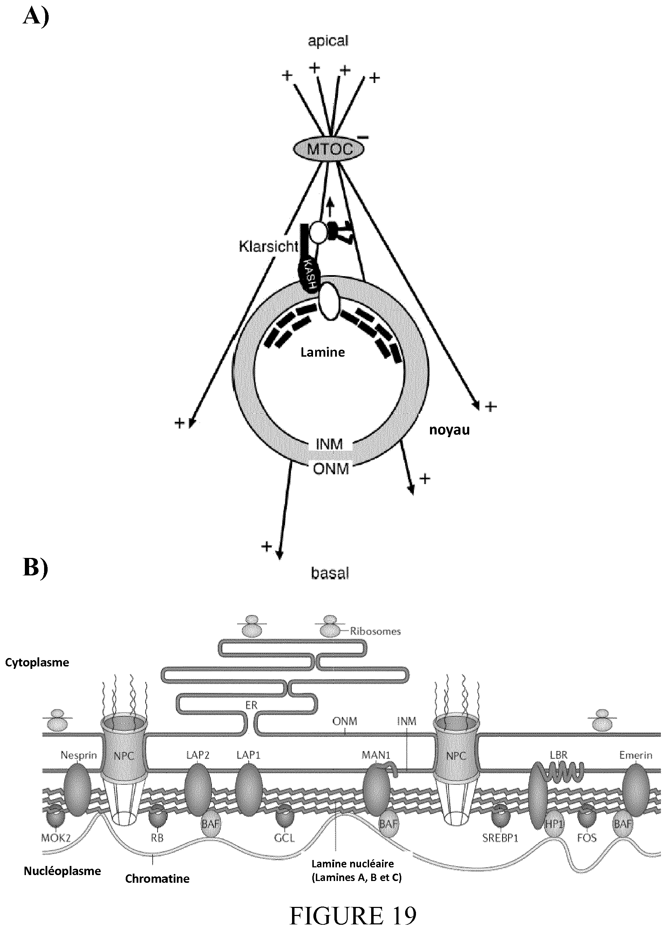

**Figure 19** **:** Modèle décrivant le rôle de *klarschit.* A) *klarsicht,* via son domaine KASH, interagit avec la lamine, localisée sur la surface interne de la membrane nucléaire (d'après : Patterson et al., 2004). B) Vue schématique montrant la structure et la fonction des lamines nucléaires. Les lamines sont localisées sur la surface interne de l'enveloppe nucléaire et servent au maintien de la stabilité du noyau, organisent la chromatine et lient les pores nucléaires (NPC). Plusieurs protéines interagissant avec les lamines sont également schématisées (d'après : Coutinho et al., Immunity & Ageing, 2009).

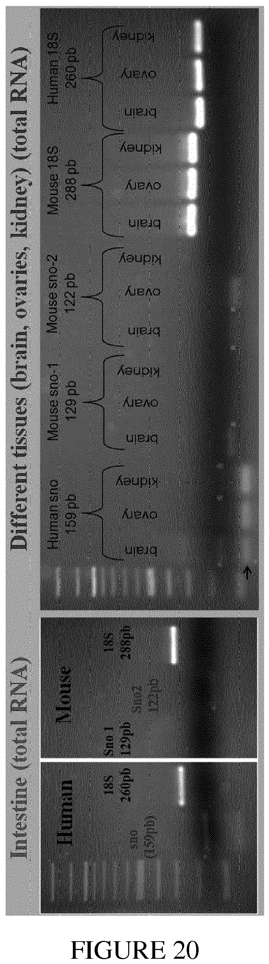

**Figure 20** **:** Les séquences orthologues de mammifères du snoARN *jouvence* sont exprimées chez la souris et l'humain (Détection par RT-PCR des homologues de souris et d'humain). L'expression de ces snoARN indique que ces derniers sont très vraisemblablement fonctionnels. Chez l'humain, le snoARN de159 bp (SEQ ID NO : 2) est exprimé dans l'intestin, le cerveau, et faiblement dans les ovaires et les reins (marqué par un astérisque rouge pour plus de précision). Chez la souris, le snoARN-1 de 129 bp (SEQ ID NO :3) est exprimé dans le cerveau uniquement. Par contre, le snoARN-2 de 122 bp (SEQ ID NO :4) est exprimé dans l'intestin, le cerveau et les ovaires, mais pas dans les reins.

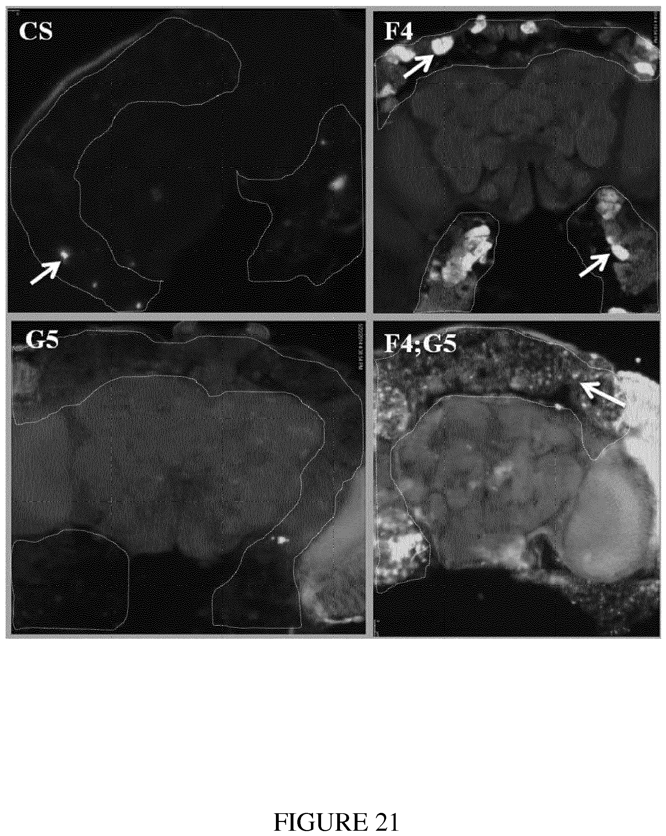

**Figure 21** **:** Les mouches mutées pour le snoARN *jouvence* (mutant F4) présentent une hypertrophie du corps gras. Des altérations importantes de ce dernier sont observées chez les vieilles mouches âgées de 40 jours, comparées à leurs contrôles respectifs, toutes âgées également de 40 jours. Ces lésions sont mises en évidence par un marquage avec un anticorps anti-caspase-3 activé, qui marque les cellules en apoptose. A) Mouches contrôles « CS ». Le corps gras, entouré d'un trait en pointillé blanc, est plutôt assez homogène et lisse. B) Mouches mutantes F4 : on remarque de gros agrégats de cellules (flèche blanche). C) Chez les mouches portant le transgène snoARN (G5) : le corps gras, sans agrégat, ressemble au corps gras contrôle « CS ». D) Chez les mouches portant le transgène G5 dans le fond génétique mutant F4 (F4;G5), ces mouches ne montrent que quelques agrégats, et ces derniers sont de petites tailles, ce qui indique que le transgène sauve, en partie, les lésions du corps gras dues à la mutation. Ces résultats suggèrent fortement une perturbation du métabolisme des glucides et/ou des lipides.

### PARTIE EXPERIMENTALE

### 1) Caractérisation génétique et moléculaire du snoARN :Ψ28S-1153 (jouvence) localisé dans le locus P[GAL4]4C.

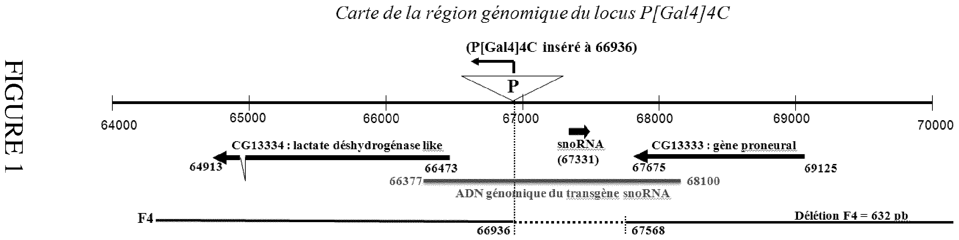

Cette expérience s'inscrit dans le cadre de la recherche des bases neurales impliquées dans le comportement locomoteur chez la Drosophile et de l'étude de la relation entre la structure et la fonction du complexe central et plus particulièrement du corps ellipsoïdal. Dans ce contexte, le criblage d'une librairie de lignées enhancer-trap P[GAL4] a permis d'identifier la lignée P[GAL4]4C qui s'exprime spécifiquement dans le corps ellipsoïdal (Figure 1). Différentes approches génétiques, notamment l'expression ciblée de la toxine tétanique, a permis de montrer que le blocage de ces neurones engendrent des défauts dans l'activité locomotrice (Martin et al., 2002). Dans un deuxième temps, afin de caractériser plus en détails ces neurones et de mieux déterminer leur fonction au sein du réseau neuronal impliqué dans l'activité locomotrice, le locus d'insertion de la lignée P[GAL4]4C a été caractérisé sur les plans génétique et moléculaire. Un PCR-rescue a été réalisé et a permis de mettre en évidence le point d'insertion de l'élément-P du P[GAL4]4C sur le chromosome 2R (Right : c'est-à-dire sur le bras droit), en position 50A, entre deux gènes : CG13333 et CG13334 (Figure 1). Ensuite, afin de pouvoir révéler le phénotype et la fonction respective de ces deux gènes, des mutations de ces derniers ont été générées par re-excision de l'élément-P (par une approche génétique appelée: jump-out, ou revertant). Une petite délétion de 632 paires de base (pb) nommée F4 a ainsi été obtenue (Figure 1).
Le snoARN:Ψ28S-1153 a été pré-identifié dans ce locus en position 67331 (Figure 1) dans le cadre du criblage systématique réalisé par Huang et al. (2005) de tous les small nucleolar RNA (snoARN) potentiels dans le génome de la Drosophile. L'article correspondant ne distingue toutefois aucun snoARN particulier et n'associe surtout aucune fonction au snoARN:Ψ28S-1153. Dans le cadre de la présente invention, ce snoARN, absent de la petite délétion F4, a été précisément positionné et caractérisé sur les plans structurel et fonctionnel. Ce snoARN est ainsi constitué de 148 paires de bases (pb) et sa structure comporte une boîte H/ACA (H/ACA box).

### 2) Caractérisation phénotypique de la délétion F4 : réduction de la durée de vie.

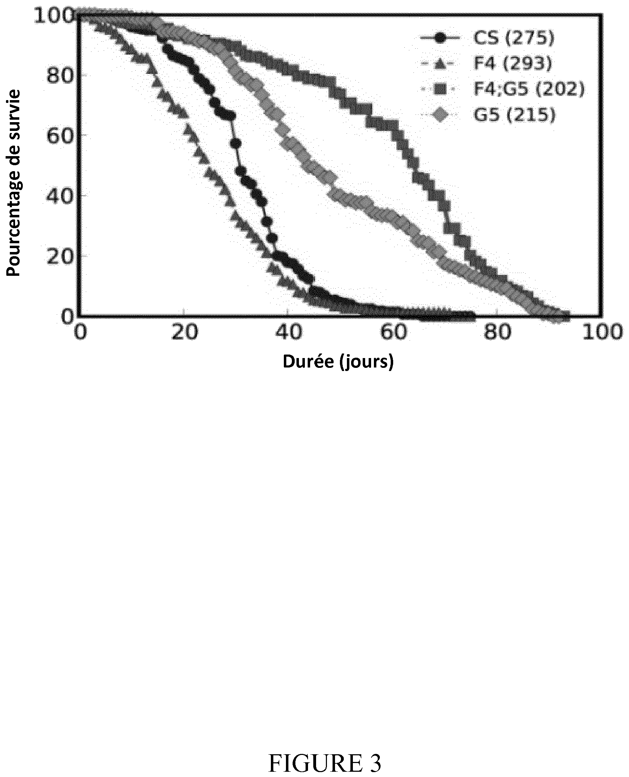

En parallèle de l'étude effectuée pour quantifier l'activité locomotrice des mouches, via l'expression ciblée de la toxine tétanique dans les ring-neurons marqués de la lignée P[GAL4] 4C, l'inventeur a observé que ces mouches ont une durée de vie très réduite et a décidé de quantifier précisément ladite durée de vie des mouches P[GAL4]4C/UAS-tetanus-toxin, ainsi que celle des mouches F4 (mutées dans le locus 4C). Ainsi, les mouches F4 présentent une duré de vie réduite (d'environ 30% par rapport aux mouches contrôles de type sauvage), suggérant que la délétion du snoARN pourrait affecter la durée de vie (longévité) (Figure 3). Les inventeurs ont aussi observé que cet effet est différent selon le sexe des mouches, l'effet étant beaucoup plus prononcé chez les femelles que chez les mâles.

### 3) Genèse d'une lignée transgénique contenant la région génomique du snoARN (jouvence) dans le but de restaurer le phénotype sauvage (« sauvetage » ou « rescue »).

Afin de démonter que le phénotype du mutant F4 est bien dû à la délétion du snoARN, une lignée de Drosophile transgénique comportant une séquence d'ADN génomique de 1723 bp de la région (de 66377 à 68100), comprenant le snoARN (Figure 1) et ses séquences régulatrices, a été générée. Plus précisément, un fragment de l'ADN génomique de la région de 1723 bp a été amplifié par PCR et inséré, via le site de restriction Xba1 dans le vecteur pCaSper-4 (ce dernier ne contenant aucune séquence promotrice/régulatrice). Les mouches transgéniques ont ensuite été générées selon une technique standard et des lignées de mouches exprimant le même transgène, mais inséré dans des endroits distincts du génome ont été obtenues (insertions indépendantes : G4, G5).
Par la suite, afin de vérifier si le transgène est fonctionnel et peut sauver (rescuer) la mutation F4 (c'est-à-dire restaurer le phénotype sauvage, à savoir restaurer une durée de vie équivalente à celle observée sans délétion F4), l'inventeur a introduit ces lignées transgéniques dans le contexte génétique mutant F4, par des croisements génétiques standards (F4;G4 et F4;G5). Il a ainsi pu être démontré que le transgène peut sauver le phénotype dû à la mutation responsable de la diminution de la durée de vie (Figure 3) (cf. par exemple : F4 versus F4;G5). De plus, ce transgène placé dans un génome de type sauvage (normal) (ce qui correspond à une sur-expression de ce snoARN, puisqu'il y a maintenant 4 copies du snoARN au lieu de deux copies endogènes) (G5) augmente la durée de vie (voir double la durée de vie dans le cas de F4;G5 ou augmente d'environ 30 % pour G5) (Figure 3). Cette expérience démontre ainsi clairement que l'on peut augmenter la durée de vie de l'animal en sur-exprimant ce snoARN (ou en modifiant son niveau d'expression). En résumé, une petite délétion génomique de la région (632 paires de base, nommé F4) (Figure 1), correspondant ou équivalent à une mutation de ce gène, raccourcie la durée de vie, alors que la sur-expression (via un transgène contenant l'ADN génomique de ce snoARN) par thérapie génique non seulement sauve la mutation mais rallonge même de façon spectaculaire (double) la durée de vie du sujet traité (Figure 3).

### 4) Tests de résistance au stress

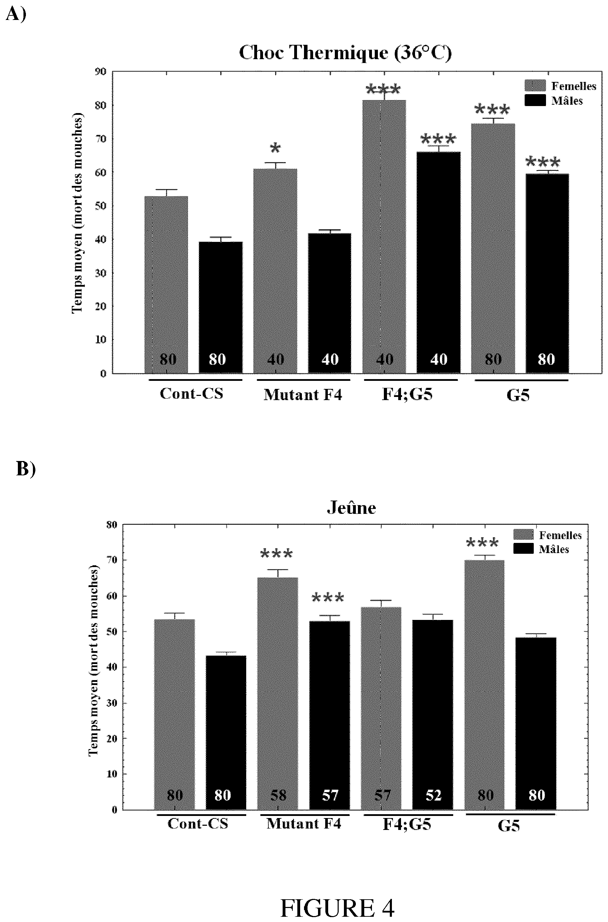

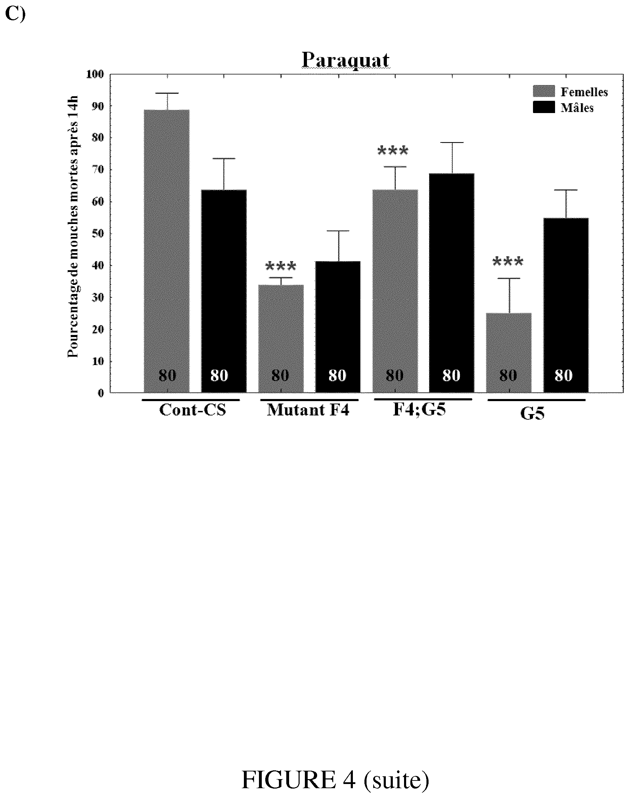

Il est maintenant bien admis que les gènes agissant sur la longévité augmentent généralement également la résistance au stress. L'inventeur a vérifié et obtenu la confirmation que la sur-expression de ce snoARN est effectivement en mesure d'augmenter la durée de vie du sujet concerné dans des conditions de stress, comme le jeûne, un choc thermique et un stress oxydatif (provoqué par le paraquat) (Figure 4).

### A) test de résistance à la chaleur (choc thermique : heat-shock test).

Les males et les femelles sont élevés ensemble dans des tubes standards contenant de la nourriture pendant 3 jours. A l'âge de 3 jours, les males et les femelles sont distribués séparément par groupe de 20 dans un tube standard contenant de la nourriture. Pour soumettre les mouches à un choc thermique (heat-shock), les tubes contenant les mouches sont placés dans un incubateur à 36°C. Le nombre de mouches mortes est compté toutes les 6 heures. Pour le choc thermique (36°C), sur la Figure 4A, on remarque que les mouches mutantes F4 (femelles seulement) sont plus résistantes que les mouches contrôles. Cependant, les mouches G5 (mâles et femelles) sont plus résistantes que les contrôles et le mutant F4, alors que du fait de l'expression du transgène G5 dans le mutant F4 (les mouches F4;G5) sont également beaucoup plus résistantes que les mouches contrôles et les mouches mutantes F4.

### B) test de jeûne

Comme pour le test de résistance à la chaleur, les males et les femelles sont élevés ensemble dans des tubes standards contenant de la nourriture pendant 3 jours. A l'âge de 3 jours, les males et les femelles sont distribués séparément par groupe de 20 dans un tube contenant seulement un papier filtre et 400 µL d'eau afin d'éviter la dessiccation. Les mouches sont gardées dans une pièce humide à 24°C, et le nombre de mouches mortes est compté toutes les 6 heures. Pour le jeûne, sur la Figure 4B, on remarque que les mouches mutantes F4 (mâles et femelles) sont plus résistantes que les mouches contrôles. De plus, les mouches G5 (femelles) sont encore plus résistantes, alors que l'expression du transgène dans le mutant F4 (F4;G5) restaure une survie normale (chez les femelles).

### C) résistance au stress oxidatif (Paraquat)

Le Paraquat (1,1'-dimethyl-4,4'-bipyridinium dichloride) réduit le NADH, ce qui génère des radicaux stables de paraquat, qui réagissent avec l'oxygène pour générer des ROS (reactive oxygen species). Conséquemment, les ROS causent des dommages aux cellules (Rzezniczak et al., 2011). Comme pour les deux tests précédents, les males et les femelles sont élevés ensemble dans des tubes standards contenant de la nourriture pendant 3 jours. A l'âge de 3 jours, les males et les femelles sont distribués séparément par groupe de 20 dans un tube standard vide, afin de les faire jeûner pendant 6 heures. Par la suite, les mouches sont transférées dans un tube contenant un papier filtre et 450µl de Paraquat, 20mM dilué dans du sucrose à 1% sucrose pour favoriser la prise alimentaire. Les mouches sont gardées dans une pièce humide à 24°C, et le nombre de mouches mortes est compté toutes les 6 heures.
Pour le stress oxydatif, sur la Figure 4C, on remarque que les mouches mutantes F4 (femelles seulement, bien qu'il y a une forte tendance aussi pour les mâles) sont plus résistantes que les mouches contrôles. De façon similaire, les mouches femelles G5 sont plus résistantes que les contrôles et similaire aux mutants F4, alors que l'expression du transgène dans le mutant F4 (F4;G5) restaure la survie des mouches (équivalent aux mouches contrôles).
En résumé, les mouches mutantes F4 sont plus résistantes que les mouches contrôles, alors que la sur-expression (G5) augmente encore cette résistance (un effet plus marqué et consistant chez les femelles que chez les mâles). De plus, dans deux tests (jeûne et stress oxydatif), l'expression du transgène dans le mutant F4 (F4;G5) restaure, en particulier chez les femelles, la survie des mouches. Ainsi, contrairement à la longévité ou la mutation F4 diminue la durée de vie, la mutation F4 augmente la résistance aux trois tests de stress effectués chez les mouches jeunes âgées de trois jours, et cet effet peut être restauré par le transgène génomique du snoARN (dans deux tests). En conclusion, la modulation de l'expression (suppression, diminution ou augmentation) du snoARN *« jouvence »* modifie la durée de vie des sujets testés.

### 5) Détermination du profil d'expression spatio-temporel du snoARN (hybridation in situ)

Afin de déterminer dans quelles cellules et/ou tissus est exprimé et agit le snoARN d'intérêt, le profil d'expression spatio-temporel de ce snoARN a été déterminé dans le cadre de l'invention, chez la mouche adulte, par hybridation *in situ* (HIS), à l'aide d'une sonde anti-sens snoARN. La tyramide a été utilisée afin d'amplifier le marquage. Tant chez les mâles que chez les femelles, le snoARN *jouvence* est exprimé dans la paroi (épithélium) de l'intestin (Figure 5A), alors que tel qu'attendu, il est absent chez le mutant F4 (Figure 5B). Plus spécifiquement, l'utilisation de lignées P[Gal4] spécifique des divers type cellulaires de l'intestin, jumelé à des double marquages, a permis de démontrer que le snoARN *jouvence* est exprimé spécifiquement dans le nucléole des entérocytes, les cellules principales et majoritaires formant l'épithélium intestinale (Figure 6A), alors qu'il n'est pas exprimé dans les autres types cellulaires (Figure 6B,C,D,E). De plus, chez les femelles, il est également exprimé dans les ovaires, et plus particulièrement dans les cellules nourricières (nurse cells) (Figures 5C et 5D).

### 6) Expression ciblée du snoARN dans les autres types cellulaires de l'épithélium de l'intestin.

L'expression ciblée (ectopique) du snoARN (UAS-8M) dans les autres types cellulaires de l'épithélium de l'intestin via l'utilisation des trois autres lignées pilotes Gal4 distinctes a été effectuée : esg-Gal4 (esg-Gal4,F4/F4;UAS-8M/+) et Delta-Gal4 (Delta-Gal4,F4/F4;UAS-8M/+) ciblant les cellules souches intestinales (ISCs), alors que Su(H)-Gal4 (Su(H)-GBE-Gal4, F4/F4 ; UAS-8M/+) cible les entéroblastes (Figure 7). Ces résultats démontrent : 1) que le snoARN peut être exprimé de façon ectopique dans d'autres types cellulaires, 2) que cette expression ectopique augmente aussi la résistance à certains stress (Figure 8). En résumé, ces résultats démontrent que le snoARN peut également conférer une protection lorsqu'il est exprimé dans les autres types cellulaires de l'épithélium de l'intestin.

### 7) Expression ciblée (sélective) du snoARN conduisant à un sauvetage de la résistance aux stress.

L'inventeur a démontré, par deux approches indépendantes, qu'une expression ciblée dans les cellules de l'intestin est suffisante pour restaurer le phénotype de résistance aux stress. Il a pu être démontré, par la technique d'hybridation *in situ*, pratiquée à partir des lignées transgéniques génomiques (celles qui augmentent la longévité : G5, ainsi qu'avec l'autre insertion indépendante, G4) que le snoARN est exprimé dans les cellules intestinales et que, pour la régulation de la longévité, l'expression du snoARN dans les cellules intestinales suffit.
Afin de confirmer ce premier résultat, le snoARN a été ciblé uniquement dans les cellules intestinales, en utilisant le système binaire d'expression P[Gal4]. Un vecteur plasmidique (p[UAS-snoARN]) a été construit dans lequel le snoARN d'intérêt (seulement les 148 pb) est placé sous le contrôle des éléments régulateurs (Upstream Activating Sequence : UAS) du Gal4. Des lignées de mouches transgéniques (UAS-snoRNA : 4M, 5M et 8M) ont ensuite été générées. Pour l'expression ciblée, les lignées dites « pilotes » de mouches transgéniques contenant un transgène (pChs-Gal4) (lignées de vecteurs plasmidiques Myo1A-Gal4, esg-Gal4 (escargot-Gal4), Su(H)GBE-Gal4, et Dl-Gal4 (Delta-Gal4)), connues pour s'exprimer en particulier dans les cellules intestinales, ont été utilisées (Jiang and Edgar, 2011; Takashima et al., 2011). Par la suite, ces divers transgènes ont été placés dans le fond génétique mutant F4, et il a été démontré, par hybridation *in situ*, que le snoARN est bien exprimé dans ces divers types de cellules intestinales (Figures 6 et 7) suite à son expression ciblée (esg-Gal4,F4/F4;UAS-snoARN-8M) (Myo1A-Gal4,F4/F4 ;UAS-snoARN-8M) (Su(H)-GBE-Gal4,F4/F4 ;UAS-snoARN-8M) (F4/F4; Delta-Gal4,/UAS-snoARN-8M). Les résultats montrent que cette expression ciblée (dans le mutant F4) restaure en substance le phénotype de résistance au stress (jeûne et choc thermique) (Figure 8).
Ces expériences démontrent clairement que la manipulation de ce snoARN dans les cellules intestinales, et plus spécifiquement dans les entérocytes, est nécessaire et suffisante pour restaurer la résistance au stress. Ces expériences, bien que conduites chez la Drosophile, permettent d'avancer que la reexpression (restauration de l'expression ou sauvetage) ou la sur-expression du snoARN dans les cellules épithéliales de l'intestin est en mesure de conduire à une augmentation de la durée de vie chez les mammifères et en particulier chez l'humain.

### 8) Identification d'homologues de la séquence jouvence chez d'autres espèces de drosophile et chez des mammifères dont la souris et l'être humain.

Des recherches par homologie de séquences ont montré que ces snoARN existent aussi chez les 11 autres espèces de Drosophiles dont le génome est disponible (Figure 9A) (voir aussi la structure consensus des 12 espèces de Drosophile : Figure 9B).
De plus, une recherche par homologie et surtout via la structure de l'ARN (utilisant le software « INFERNAL ») a permis d'identifier un homologue chez l'humain, localisé sur le chromosome 11, en position : 12822722-12822811 (Figure 10). Chez la souris, deux homologues ont aussi été identifiés: sur le chromosome 15, position: 30336889-30337017; et sur le chromosome 18, position : 6495012-6495091 (Figures 11A et 11B).
Comme chez la Drosophile, il est très probable que la sur-expression de ce gène et/ou d'un analogue synthétique (soit génétiquement, soit par l'administration orale, soit par injection) rallonge la durée de vie chez l'humain. Une telle expression est également en mesure de protéger contre les effets délétères des diverses maladies dégénératives.

### 9) Rôle du snoARN dans la neuro-dégénérescence et la neuro-protection

Les mouches F4 ayant une durée de vie diminuée, alors que les mouches qui sur-expriment le snoARN (G5) vivent plus longtemps, les vieilles mouches (âgées de 40 jours) ont été étudiées afin de vérifier la présence éventuelle, chez elles, de lésions de neuro-dégénérescence (présence de trous plus ou moins nombreux et de taille plus ou moins importante dans le cerveau). Globalement, chez les mouches contrôles (Canton-S), environ 60% des mouches présentent des lésions (mais soulignons que 50 % des mouches CS sont déjà mortes à 40 jours) (Figure 12) [pour plus de précision : voir la Figure 12d pour une analyse semi-quantitative]. Chez les mutants F4, toutes les mouches (100%) présentent des lésions, et ces dernières sont généralement plus sévères que celles des contrôles (taille et nombre de trous), alors que les mouches F4;G5 présentent nettement moins de trous (environ seulement 10% des mouches), ces derniers étant par ailleurs de taille moins importante. Finalement, chez les mouches G5, environ le même pourcentage de mouches que les contrôles CS présentent des lésions (environ 40%) mais ces lésions sont nettement moins sévères (Figure 12d). En résumée, le transgène génomique (F4;G5) sauve (en partie) le phénotype de neuro-dégénérescence, alors que la sur-expression du snoARN (G5) protège contre la neuro-dégénérescence.

### 10) Rôle du snoARN dans la protection des paramètres sensori-moteurs.

De façon similaire, afin de rechercher si la neuro-protection procurée par le snoARN a des effets sur certains paramètres physiologiques tels que les paramètres sensori-moteurs, l'activité locomotrice des mouches âgées de 40 jours a été quantifiée (par video-tracking) (Martin, 2004) puis comparée à celles des mouches jeunes, âgées de 4 jours (Figure 13). D'abord, une énorme différence entre les mouches âgée de 4 jours et celles âgées de 40 jours est observée ; les mouches de 40 jours parcourent une distance environ trois fois inférieure à celle parcourue par les mouches âgées de 4 jours. Cependant, chez les vieilles mouches âgées de 40 jours, les mouches qui sur-expriment le snoARN (G5) marchent plus que les mouches contrôles (Canton-S), mutantes F4, et celles exprimant le snoARN dans le fond génétique mutant (F4;G5). Cet effet est plus marqué chez les mâles que chez les femelles. En résumé, sur les vieilles mouches âgées de 40 jours, on voit que les mouches sur-exprimant le snoARN présentent de meilleures performances sensori-motrices que les mouches contrôles. Ces résultats démontrent le caractère protecteur du snoARN vis-à-vis des effets délétères associés aux mécanismes du vieillissement.

### 11) Recherche de la fonction du snoARN dans les ovaires : rôle sur la fertilité (reproduction)

Chez les femelles, le snoARN s'exprime également dans les ovaires et plus précisément dans les cellules nourricières (Figures 5C,D). Les inventeurs ont également montré que les mouches mutantes (F4) présentent aussi des modifications de fertilité (phénotype reproducteur). Les mouches F4 pondent un peu plus d'oeuf que les mouches contrôles (CS) (Figure 14). Les mouches portant le snoARN dans le fond génétique mutant (F4;G4) pondent beaucoup moins d'oeuf (fertilité diminuée), alors que les mouches sur-exprimant le snoARN (G4) pondent beaucoup plus d'oeufs que les mouches contrôles (Figure 14). Ces résultats démontrent indéniablement l'effet important exercé par le snoARN dans les ovaires et son influence sur la fertilité des femelles.

### 12) Traitement à partir de snoARN (administration par voie orale ou injection).

Le snoARN peut être administré soit par voie orale (*per os*) soit par injection. D'autres voies d'administration telles que par exemple l'inhalation et l'application locale/cutanée sont également utilisables selon le type de vecteur utilisé.
Comme le démontrent les expériences réalisées dans le cadre de l'invention, l'administration orale du snoARN est possible compte tenu de sa stabilité et de sa résistance *in vitro* ainsi que de sa capacité à agir localement sur les cellules de la paroi intestinale (entéroblastes, enterocytes, ISCs). Comme expliqué précédemment, l'administration du snoARN d'intérêt est ainsi en mesure de permettre une expression autrement absente (ou en d'autres termes de sauver une mutation existante) ou, si besoin, de permettre une surexpression, afin de protéger la paroi intestinale vis-à-vis des agressions tout en maintenant un meilleur équilibre endocrinien et métabolique.

### 13) Traitement contre le cancer.

Tant chez les mammifères que chez la Drosophile, il a été montré que les gènes *delta*/*notch* régulent la différentiation des cellules progénitrices de l'intestin (gut), alors que la voie de signalisation de Wnt participe au maintien et à la prolifération des ISCs. De même, la voie de signalisation des cytokines (Upd/Jak/Stat) ainsi que celle de l'EGFR (Epidermal Growth Factor Receptor) régulent la prolifération des ISCs (Figures 15-16) (Jiang and Edgar, 2011; Takashima et al., 2011).
Nos expériences démontrent que le snoARN d'intérêt selon l'invention est en mesure de réguler certains gènes tels que les gènes *notch, delta, JNK, EGFR, etc.* (Figure 15) dont la dérégulation peut conduire à une hyper-prolifération de l'épithélium intestinal et donc à des cancers (Jiang and Edgar, 2011; Takashima et al., 2011). Le snoARN d'intérêt selon l'invention est en particulier en mesure de prévenir ou de traiter le cancer grâce à sa capacité à réguler l'expression de l'EGFR, la voie de signalisation de l'EGFR dans la régulation de la prolifération des ISCs dans l'intestin de la Drosophile et des mammifères étant en effet particulièrement bien conservée (Figure 16) [plusieurs thérapies sont actuellement en cours d'essaie clinique pour le traitement du cancer du colon, dont notamment deux anticorps monoclonal anti-EGFR (*Cetuximab et Panitumumab*) (Amado et al., 2008; Di Nicolantonio et al., 2008)].

### 14) Implication du snoARN d'intérêt (« jouvence ») dans le syndrome de Hutchinson-Gilford (HGPS), connu aussi sous le nom de progéria, qui consiste en un vieillissement précoce et accéléré de l'être humain.

Nous avons démontré que le snoARN *jouvence* régule l'épissage (quantité d'ARN épissé) du gène *klarsicht* (Figure 18A). Il a été montré que ce dernier interagit avec la lamine (Patterson et al., 2004), une protéine localisée sous la membrane interne du noyau et impliquée dans la progéria (maladie engendrant un vieillissement précoce et accéléré de l'être humain) (Figure 18B). La progéria est une maladie rare touchant environ 1 individu sur 8 millions (Scaffidi and Misteli, 2006 ; Broers et al., 2006 ; Cohen et al., 2001 ; Cau et al., 2014 ; Coutinho et al., 2009). Le snoARN *jouvence* offre, de par son phénotype (implication dans la longévité) et son implication dans la régulation du gène *klarsicht,* une nouvelle perspective de traitement des laminopathies telles que le syndrome de Hutchinson-Gilford ou progéria, la dysplasie Mandibuloacral (MAD), la dystrophie musculaire de Emery-Dreifuss, le syndrome atypique de Werner, la dermatophatie restrictive, l'akinésie fetale léthale, et la LIRLLC (Generalized lipoatrophy, insulin-resistant diabetes, leukomelanodermic papules, liver steatosis, and hypertrophic cardiomyopathy) (Hutchison, 2002 ; Broers et al., 2006), en particulier du syndrome de Hutchinson-Gilford (progéria).

### 15) Implication du snoARN d'intérêt (« jouvence ») dans la régulation de l'épissage du gène fatty acid 2-hydroylase (fa2h), et en conséquence dans les diverses formes de neuro-dégénérescence associées à ce gène.

Nous avons démontré que le snoARN *jouvence* régule l'épissage (quantité d'ARN épissé) du gène CG30502 codant l'acide gras 2-hydroxylase ou « fatty acid 2-hydroxylase *»* (*fa2h*) (Figure 18B). Ce gène est impliqué dans le processus de biosynthèse des acides gras et plus particulièrement dans les processus métaboliques des lipides complexes comme les sphingolipides et les céramides (Carvalho et al., 2010). Chez l'humain, ce gène *fa2h* a été associé à diverses formes de neuro-dégénérescence (démyélinisation) comme celles associées à l'accumulation de fer dans le cerveau, certaines leucodystrophies, et finalement la paraplégie spastique héréditaire (SPG35) (Kruer et al., 2010 ; Pierson et al., 2012 ; Schneider and Bhatia, 2010). Le snoARN *jouvence* offre, de par son phénotype (implication dans la longévité et/ou l'hypertrophie du corps gras observé chez le mutant F4) et son implication dans la régulation du gène *fa2h*, une nouvelle perspective de traitement des maladies neurodégénératives mentionnées ci-dessus.

### 16) Relation métabolique et neuro-endocrinienne entre l'intestin et le cerveau (axe cerveau-intestin).

Comme mentionné précédemment, les mutants du snoARN (F4) présentent une hypertrophie du corps gras, visible tant au niveau de l'abdomen qu'autour du cerveau dans la capsule céphalique (Figure 21). De plus, la quantification des triglycérides a permis de confirmer cette hypertrophie (d'où le qualificatif de mouches dites « obèses »). Ce phénotype suggère une relation métabolique et/ou neuroendocrinienne entre l'expression du snoARN dans l'intestin, les lésions neuro-dégénératives et l'augmentation de la durée de vie. Plus spécifiquement, cette perturbation métabolique suggère une implication de la voie de signalisation de l'insuline, cette dernière ayant été mainte fois décrite comme impliquée dans la longévité des Drosophiles (Tatar et al., 2001 ; Bai et al., 2012 ; Partridge et al., 2011 ; Fontana et al., 2010). Des marquages immuno-histochimiques dirigés contre l'anti-caspase-3 activé a permis de montrer, chez les Drosophiles adultes, que le corps gras localisé au pourtour du cerveau est fortement perturbé (i.e. hypertrophié) chez les mutants F4, alors qu'il est protégé par la sur-expression du snoARN (transgènes G5) ainsi que lors du sauvetage (restauration de l'expression) (F4;G5) (Figure 21). Ces résultats démontrent que le snoARN protège l'organisme qui l'exprime des altérations du corps gras, et donc par extension de l'obésité. De plus, des expériences d'interactions génétiques (double mutants) mettant en jeu des mutations du récepteur de l'insuline (InR) et le mutant F4 montrent que la mutation du snoARN peut sauver au moins partiellement le phénotype de la mutation du récepteur de l'insuline (cf. figure 21 et sa légende). Ces résultats démontrent l'existence de liens (qu'ils soient directs ou indirects) entre la voie de signalisation de l'insuline, le snoARN, et la longévité. Les résultats précédents démontrent que la mutation et/ou la dérégulation du snoARN perturbe le métabolisme des hydrates de carbone (glucides) et des lipides.
Le snoARN *jouvence* offre ainsi une nouvelle perspective de traitement du diabète comme de l'obésité.

### 17) Expression du snoARN d'intérêt (« jouvence ») chez l'humain et la souris.

Par une approche de RT-PCR, nous avons démontré l'existence de séquences homologues, et plus précisément de séquences orthologues, au snoARN *jouvence* (d'abord identifié chez la drosophile) concrètement exprimées chez les mammifères (Figure 20). Chez l'humain, le snoARN de 159 bp (SEQ ID NO :2) s'exprime dans l'intestin, le cerveau, et faiblement dans les ovaires et les reins. Chez la souris, le snoARN-1 de129 bp (SEQ ID NO : 3) s'exprime uniquement dans le cerveau et le snoARN-2 de 122 bp (SEQ ID NO :4) s'exprime dans l'intestin, le cerveau et les ovaires, mais pas dans les reins. Ces données supportent l'expression très probablement fonctionnelle de ces snoARNs chez l'humain comme chez la souris.

### REFERENCES

- Amado RG, Wolf M, Peeters M, Van Cutsem E, Siena S, Freeman DJ, Juan T, Sikorski R, Suggs S, Radinsky R, Patterson SD, Chang DD (2008). Wild-type KRAS is required for panitumumab efficacy in patients with metastatic colorectal cancer. J Clin Oncol, 26, 1626-1634.
- Bai H, Kang P, Tatar M. (2012). Drosophila insulin-like peptide-6 (dilp6) expression from fat body extends lifespan and represses sécrétion of Drosophila insulin-like peptide-2 from the brain. Aging Cell., 11, 978-985.
- Brand AH, Perrimon N (1993) Targeted gene expression as a means of altering cell fates and generating dominant phenotypes. Development 118, 401-415
- Broers JL, Ramaekers FC, Bonne G, Yaou RB, Hutchison CJ (2006). Nuclear lamins: laminopathies and their rôle in prématuré ageing. Physiol Rev., 86, 967-1008.
- Carvalho M, Schwudke D, Sampaio JL, Palm W, Riezman I, Dey G, Gupta GD, Mayor S, Riezman H, Shevchenko A, Kurzchalia TV, Eaton S. (2010) Survival stratégies of a sterol auxotroph. Development, 137, 3675-3685.
- Cau P, Navarro C, Harhouri K, Roll P, Sigaudy S, Kaspi E, Perrin S, De Sandre-Giovannoli A, Lévy N. (2014). Nuclear matrix, nuclear envelope and prématuré aging syndromes in a translational research perspective. Semin Cell Dev Biol., 28, S1084-9521(14)00058-5.
- Cohen M, Lee KK, Wilson KL, Gruenbaum Y. (2001). Transcriptional repression, apoptosis, human disease and the functional évolution of the nuclear lamina. Trends Biochem Sci., 26, 41-47.
- Coutinho HD1, Falcâo-Silva VS, Gonçalves GF, da Nóbrega RB (2009). Molecular ageing in progeroid syndromes: Hutchinson-Gilford progeria syndrome as a model. Immun Ageing, 20, 6:4.
- Dick, K. J., Al-Mjeni, R., Baskir, W., Koul, R., Simpson, M. A., Patton, M. A., Raeburn, S., Crosby, A. H. (2008). A novel locus for an autosomal recessive hereditary spastic paraplegia (SPG35) maps to 16q21-q23. Neurology 71: 248-252.
- Dick, K. J., Eckhardt, M., Paisan-Ruiz, C., Alshehhi, A. A., Proukakis, C., Sibtain, N. A., Maier, H., Sharifi, R., Patton, M. A., Bashir, W., Koul, R., Raeburn, S., Gieselmann, V., Houlden, H., Crosby, A. H. (2010). Mutation of FA2H underlies a complicated form of hereditary spastic paraplegia (SPG35). Hum. Mutat. 31: E1251-1260.
- Di Nicolantonio F, Martini M, Molinari F, Sartore-Bianchi A, Arena S, Saletti P, De Dosso S, Mazzucchelli L, Frattini M, Siena S, Bardelli A (2008). Wild-type BRAF is required for response to panitumumab or cetuximab in metastatic colorectal cancer. J Clin Oncol, 26, 5705-5712.
- Elliott DA, Brand AH. (2008) The GAL4 system: a versatile system for the expression of genes. Methods Mol Biol, 420, 79-95.
- Fontana L, Partridge L, Longo VL (2010). Extending healthy life span - from yeast to humans. Science, 328, 321-326.
- Huang ZP, Zhou H, He HL, Chen CL, Liang D, Qu LH. (2005) Genome-wide analyses of two families of snoRNA genes from Drosophila melanogaster, demonstrating the extensive utilization of introns for coding of snoRNAs. RNA, 11, 1303-1316.
- Hutchison CJ. (2002). Lamins: building blocks or regulators of gene expression? Nat Rev Mol Cell Biol., 3, 848-858.
- Jiang H, Edgar BA (2011). Intestinal stem cells in the adult Drosophila midgut. Exp Cell Res. 317, 2780-2788.
- Kruer MC1, Paisán-Ruiz C, Boddaert N, Yoon MY, Hama H, Gregory A, Malandrini A, Woltjer RL, Munnich A, Gobin S, Polster BJ, Palmeri S, Edvardson S, Hardy J, Houlden H, Hayflick SJ. (2010). Defective FA2H leads to a novel form of neurodegeneration with brain iron accumulation (NBIA). Ann Neurol., 68, 611-618.
- Martin, JR, Faure, P, Ernst, R (2002). The Power Law Distribution for Walking-Time Intervals Correlates with the Ellipsoid Body in Drosophila. J. Neurogenetics, 15, 1-15.
- Martin, JR (2004). A portrait of locomotor behaviour in Drosophila determined by a video-tracking paradigm. Behav. Process., 67, 207-219.
- Partridge L, Alic N, Bjedov I, Piper MD (2011). Ageing in Drosophila: the role of the insulin/Igf and TOR signalling network. Exp Gerontol., 46, 376-381.
- Patterson K1, Molofsky AB, Robinson C, Acosta S, Cater C, Fischer JA. (2004) The functions of Klarsicht and nuclear lamin in developmentally regulated nuclear migrations of photoreceptor cells in the Drosophila eye. Mol Biol Cell., 15, 600-610.
- Pierson TM1, Simeonov DR, Sincan M, Adams DA, Markello T, Golas G, Fuentes-Fajardo K, Hansen NF, Cherukuri PF, Cruz P, Mullikin JC, Blackstone C, Tifft C, Boerkoel CF, Gahl WA. (2012) Exome sequencing and SNP analysis detect novel compound heterozygosity in fatty acid hydroxylase-associated neurodegeneration. Eur J Hum Genet., 20, 476-479.
- Rzezniczak TZ, Douglas LA, Watterson JH, Merritt TJ. (2011) Paraquat administration in Drosophila for use in metabolic studies of oxidative stress. Analytical Biochem., 419, 345-7.
- Scaffidi P, Misteli T. (2006) Lamin A-dependent nuclear defects in human aging. Science, 312, 1059-1063.
- Schneider SA, Bhatia KP. (2010) Three faces of the same gene: FA2H links neurodegeneration with brain iron accumulation, leukodystrophies, and hereditary spastic paraplegias. Ann Neurol., 68, 575-577.
- Takashima S, Adams KL, Ortiz PA, Ying CT, Moridzadeh R, Younossi-Hartenstein A, Hartenstein V (2011). Development of the Drosophila entero-endocrine lineage and its specification by the Notch signaling pathway. Dev Biol, 353, 161-172.
- Tatar M, Kopelman A, Epstein D, Tu MP, Yin CM, Garofalo RS (2001). A Mutant Drosophila Insulin Receptor Homolog That Extends Life-Span and Impairs Neuroendocrine Function. Science, 292, 107.
- Uytterhoeven V, Kuenen S, Kasprowicz J, Miskiewicz K, Verstreken P (2011). Loss of skywalker reveals synaptic endosomes as sorting stations for synaptic vesicle proteins. Cell, 145, 117-132.

## Revendications

1. Séquence ARN isolée ou synthétique comprenant la séquence SEQ ID NO : 1, une séquence orthologue, ou une séquence analogue sur le plan fonctionnel pour une utilisation en tant que médicament.

2. Séquence ARN pour l'utilisation selon la revendication 1, **caractérisée en ce que** ladite séquence orthologue est d'origine humaine et consiste en la séquence SEQ ID NO : 2 ou est d'origine murine et consiste en une séquence choisie parmi SEQ ID NO : 3 et SEQ ID NO : 4.

3. Séquence ARN pour l'utilisation selon la revendication 1 ou 2, **caractérisée en ce que** ladite séquence est un petit ARN nucléolaire (snoARN).

4. Séquence ARN pour l'utilisation selon la revendication 1, **caractérisée en ce que** ladite séquence analogue sur le plan fonctionnel permet l'épissage d'un gène de *Drosophila melanogaster* choisi parmi Ir56d, buttonhead, klarsicht, CG3262, CG30502 (*fa2h*), CG11125, CG9339 et CG40006.

5. Séquence ARN selon l'une quelconque des revendications 1 à 4 pour une utilisation dans la prolongation de la durée de vie d'un sujet, l'augmentation de la résistance à un stress, ou la lutte contre les effets délétères du vieillissement chez un sujet.

6. Séquence ARN selon l'une des revendications 1 à 5 pour une utilisation dans la prévention ou le traitement chez un sujet d'une maladie dégénérative, en particulier d'une maladie neurodégénérative, d'une laminopathie, du diabète, de l'obésité, ou d'un cancer.

7. Séquence ARN pour l'utilisation selon la revendication 6 dans laquelle le cancer est un cancer induit par les radicaux libres.

8. Séquence ARN selon l'une des revendications 1 à 3 pour l'utilisation du traitement de l'infertilité ou la stimulation de la fertilité d'un sujet.

9. Séquence ARN pour l'utilisation selon l'une des revendications 5 à 8, **caractérisée en ce que** le sujet est un animal ou un insecte, typiquement un mammifère, de préférence un être humain, encore plus préférentiellement un sujet n'exprimant pas la séquence ARN selon l'une des revendications 1 à 3 ou exprimant une version anormale de ladite séquence ARN, un sujet soumis à des conditions de stress telles que le jeûne, un choc thermique, ou un stress oxydatif, et/ou un sujet souffrant d'une maladie dégénérative, d'une laminopathie, de diabète, d'obésité, d'un cancer ou d'un problème de fertilité.

10. Séquence ARN pour l'utilisation selon la revendication 9, **caractérisée en ce que** le génome du sujet, n'exprimant pas la séquence d'ARN selon l'une des revendications 1 à 3 ou exprimant une version anormale de ladite séquence d'ARN, contient une séquence ADN comprenant une mutation, ladite séquence étant à l'état sauvage choisie parmi SEQ ID NO : 5, SEQ ID NO : 6, SEQ ID NO : 7 et SEQ ID NO :8 ; et ladite mutation étant de préférence choisie parmi une délétion, une substitution, ou une addition, d'une ou plusieurs paires de bases.

11. Séquence ADN codant une séquence ARN pour l'utilisation selon l'une des revendications 1 à 10.

12. Vecteur permettant l'expression d'une séquence ARN pour l'utilisation selon l'une des revendications 1 à 4, choisi par exemple parmi un plasmide, un cosmide, un vecteur viral, et un phage, comportant de préférence un promoteur et/ou des éléments régulateurs favorisant l'expression de ladite séquence d'ARN dans les cellules intestinales ou ovariennes.

13. Cellule comprenant une séquence ARN pour l'utilisation selon l'une des revendications 1 à 4 ou transformée à l'aide d'un vecteur pour l'utilisation selon la revendication 12.

14. Composition comprenant une séquence ARN selon l'une des revendications 1 à 4, un vecteur selon la revendication 12 ou une cellule selon la revendication 13 et un support acceptable sur le plan diététique ou pharmaceutique.

15. Utilisation d'une séquence ARN selon l'une des revendications 1 à 10, d'un vecteur selon la revendication 12, d'une cellule selon la revendication 13, ou d'une composition selon la revendication 14, de manière continue ou séquentielle, pour restaurer ou moduler l'expression de ladite séquence ARN *in vitro* ou *ex vivo.*

16. Souris transgénique dans le génome de laquelle l'une et/ou l'autre des séquences SEQ ID NO : 3 et SEQ ID NO : 4 a été modifiée pour prévenir ou modifier l'expression de l'une et/ou l'autre desdites séquences.

## Patentansprüche

1. Synthetische oder isolierte RNA-Sequenz, umfassend die Sequenz SEQ ID NO: 1, eine orthologe Sequenz oder eine funktionelle analoge Sequenz für eine Verwendung als Medikament.

2. RNA-Sequenz für die Verwendung gemäß Anspruch 1, **dadurch gekennzeichnet, dass** besagte orthologe Sequenz humanen Ursprungs ist und aus der Sequenz SEQ ID NO: 2 besteht oder murinen Ursprungs ist und aus der Sequenz besteht, ausgewählt aus SEQ ID NO: 3 und SEQ ID NO: 4.

3. RNA-Sequenz für die Verwendung gemäß Anspruch 1 oder 2, **dadurch gekennzeichnet, dass** besagte Sequenz eine kleine nukleoläre RNA (snoRNA) ist.

4. RNA-Sequenz für die Verwendung gemäß Anspruch 1, **dadurch gekennzeichnet, dass** besagte funktionelle analoge Sequenz das Spleißen eines Gens von *Drosophila melanogaster* erlaubt, das aus lr56d, buttonhead, klarsicht, CG3262, CG30502 (*fa2h*), CG11125, CG9339 und CG40006 ausgewählt ist.

5. RNA-Sequenz gemäß einem der Ansprüche 1 bis 4 für eine Verwendung bei Verlängerung der Lebenszeit eines Lebewesens, Erhöhung der Stressresistenz oder Bekämpfung schädlicher Auswirkungen der Alterung bei einem Lebewesen.

6. RNA-Sequenz gemäß einem der Ansprüche 1 bis 5 für eine Verwendung bei Prävention oder Behandlung einer degenerativen Krankheit, insbesondere einer neurodegenerativen Krankheit, einer Laminopathie, des Diabetes, der Obesität oder eines Krebses.

7. RNA-Sequenz für die Verwendung gemäß Anspruch 6, wobei der Krebs ein von freien Radikalen induzierter Krebs ist.

8. RNA-Sequenz gemäß einem der Ansprüche 1 bis 3 für die Verwendung bei Behandlung der Infertilität oder Stimulierung der Fertilität eines Lebewesens.

9. RNA-Sequenz für die Verwendung gemäß einem der Ansprüche 5 bis 8, **dadurch gekennzeichnet, dass** das Lebewesen ein Tier oder ein Insekt, üblicherweise ein Säuger, bevorzugt ein Mensch, noch bevorzugter ein Lebewesen, das die RNA-Sequenz gemäß einem der Ansprüche 1 bis 3 nicht exprimiert oder eine anormale Version besagter RNA-Sequenz exprimiert, ein Lebewesen, das Stresszuständen wie Fasten, thermischem Schock oder oxidativem Stress ausgesetzt ist, und/oder ein Lebewesen ist, das an einer degenerativen Krankheit, einer Laminopathie, Diabetes, Obesität, einem Krebs oder einem Fertilitätsproblem leidet.

10. RNA-Sequenz für die Verwendung gemäß Anspruch 9, **dadurch gekennzeichnet, dass** das Genom des Lebewesens, das die RNA-Sequenz gemäß einem der Ansprüche 1 bis 3 nicht exprimiert oder eine anormale Version besagter RNA-Sequenz exprimiert, eine Mutation umfassende DNA-Sequenz enthält, wobei besagte Sequenz als Wildtyp aus SEQ ID NO: 5, SEQ ID NO: 6, SEQ ID NO: 7 und SEQ ID NO: 8 ausgewählt ist; und besagte Mutation bevorzugt aus einer Deletion, einer Substitution oder einer Addition eines oder mehrerer Basenpaare ausgewählt ist.

11. DNA-Sequenz, die eine RNA-Sequenz für die Verwendung gemäß einem der Ansprüche 1 bis 10 codiert.

12. Vektor, der die Expression einer RNA-Sequenz für die Verwendung gemäß Anspruch 1 bis 4 erlaubt, aus einem Plasmid, einem Cosmid, einem viralen Vektor und einem Phagen ausgewählt ist und bevorzugt einen Promotor und/oder regulatorische Elemente umfasst, welche die Expression besagter RNA-Sequenz in Intestinal- oder Ovarialzellen erleichtern.

13. Zelle, die eine RNA-Sequenz für die Verwendung gemäß einem der Ansprüche 1 bis 4 umfasst oder mit Hilfe eines Vektors für die Verwendung gemäß Anspruch 12 transformiert ist.

14. Zusammensetzung, die eine RNA-Sequenz gemäß einem der Ansprüche 1 bis 4, einen Vektor gemäß Anspruch 12 oder eine Zelle gemäß Anspruch 13 und einen diätetisch oder pharmazeutisch verträglichen Träger umfasst.

15. Kontinuierliche oder sequentielle Verwendung einer RNA-Sequenz gemäß einem der Ansprüche 1 bis 10, eines Vektors gemäß Anspruch 12, einer Zelle gemäß Anspruch 13 oder einer Zusammensetzung gemäß Anspruch 14, zur Wiederherstellung oder Modulation der Expression besagter RNA-Sequenz *in vitro* oder *ex vivo.*

16. Transgene Maus, in deren Genom eine und/oder beide Sequenzen SEQ ID NO: 3 und SEQ ID NO: 4 modifiziert worden sind, um die Expression von einer und/oder beiden besagten Sequenzen zu verhindern oder zu modifizieren.

## Claims

1. An isolated or synthetic RNA sequence comprising sequence SEQ ID NO: 1, an orthologous sequence or a functionally-analogous sequence thereof, for use as a medicament.

2. The RNA sequence for use according to claim 1, **characterized in that** said orthologous sequence is of human origin and consists of sequence SEQ ID NO: 2 or is of mouse origin and consists of a sequence selected from SEQ ID NO: 3 and SEQ ID NO: 4.

3. The RNA sequence for use according to claim 1 or 2, **characterized in that** said sequence is a small nucleolar RNA (snoRNA).

4. The RNA sequence for use according to claim 1, **characterized in that** said functionally-analogous sequence permits splicing a *Drosophila melanogaster* gene selected from Ir56d, buttonhead, klarsicht, CG3262, CG30502 (*fa2h*), CG11125, CG9339 and CG40006.

5. The RNA sequence according to any one of claims 1 to 4 for use in extending the lifespan of a subject, increasing stress resistance, or fighting the harmful effects of aging in a subject.

6. The RNA sequence according to one of claims 1 to 5 for use in preventing or treating a degenerative disease in a subject, in particular a neurodegenerative disease, a laminopathy, diabetes, obesity or cancer.

7. The RNA sequence for use according to claim 6 wherein the cancer is a cancer induced by free radicals.

8. The RNA sequence according to one of claims 1 to 3 for use in treating infertility or stimulating fertility in a subject.

9. The RNA sequence for use according to one of claims 5 to 8, **characterized in that** the subject is an animal or an insect, typically a mammal, preferably a human being, even more preferentially a subject not expressing the RNA sequence according to one of claims 1 to 3 or expressing an abnormal version of said RNA sequence, a subject experiencing conditions of stress such as fasting, heat shock, or oxidative stress, and/or a subject suffering from a degenerative disease, a laminopathy, diabetes, obesity, cancer or fertility problems.

10. The RNA sequence for use according to claim 9, **characterized in that** the genome of the subject, not expressing the RNA sequence according to one of claims 1 to 3 or expressing an abnormal version of said RNA sequence, contains a DNA sequence comprising a mutation, said sequence being a wild-type sequence selected from SEQ ID NO: 5, SEQ ID NO: 6, SEQ ID NO: 7 and SEQ ID NO: 8; and said mutation being preferably selected from a deletion, a substitution, or an addition, of one or more base pairs.

11. A DNA sequence encoding an RNA sequence for use according to one of claims 1 to 10.

12. A vector permitting expression of an RNA sequence for use according to one of claims 1 to 4, selected for example from a plasmid, a cosmid, a viral vector, and a phage, preferably comprising a promoter and/or regulatory elements promoting the expression of said RNA sequence in intestinal or ovarian cells.

13. A cell comprising an RNA sequence for use according to one of claims 1 to 4 or transformed using a vector for use according to claim 12.

14. A composition comprising an RNA sequence according to one of claims 1 to 4, a vector according to claim 12 or a cell according to claim 13 and a dietarily or pharmaceutically acceptable support.

15. Use of an RNA sequence according to one of claims 1 to 10, a vector according to claim 12, a cell according to claim 13, or a composition according to claim 14, continuously or sequentially, to restore or modulate the expression of said RNA sequence *in vitro* or *ex vivo.*

16. A transgenic mouse in the genome of which one and/or the other of sequences SEQ ID NO: 3 and SEQ ID NO: 4 has been modified to prevent or modify the expression of one and/or the other of said sequences.
